# EUROPEAN PATENT APPLICATION

(11) **EP 4 286 515 A1**
(43) Date of publication of application: **06.12.2023**
(21) Application number: 22746252.0
(22) Date of filing: 27.01.2022
(51) Int. Cl.: C12N 9/10, C12N 9/00, C12N 9/12, C12N 9/02, C12N 15/70, C12P 19/04

(54) **C-GLYCOSYLTRANSFERASE VARIANTS AND USE THEREOF**

(30) Priority: 27.01.2021 KR 20210011326; 26.01.2022 KR 20220011630
(71) Applicant: Korea Advanced Institute of Science and Technology, Daejeon 34141 (KR)
(72) Inventor: LEE, Sang Yup, Daejeon 34141 (KR); YANG, Dongsoo, Daejeon 34141 (KR); JANG, Woo Dae, Daejeon 34141 (KR)
(74) Representative: Mewburn Ellis LLP
(86) International application number: PCT/KR2022/001485
(87) International publication number: WO 2022/164226

(57) **Abstract**

The present invention relates to novel C-glycosyltransferase variants and a use thereof. The C-glycosyltransferase variants according to the present invention have improved glycosidic bond-forming ability as compared with wild-type C-glycosyltransferase, and thus can increase the glycoside production effects of polyketide groups and pseudo-natural products, particularly type I, II, III polyketide, nonribosomal peptides, phenylpropanoids, and other aromatic natural products, and thus can be useful for the preparation of a drug, a food additive, a nutritional supplement, and the like containing a C-glycoside compound as a constituent ingredient.

## Description

### [Technical Field]

The present invention relates to a novel C-glycosyltransferase variant and use thereof, and more particularly to a C-glycosyltransferase variant in which glycosylation reaction of substrate carbon is enhanced due to amino acid mutation at the active site of C-glycosyltransferase, and the use thereof in production of polyketide glycosides and phenylpropanoid glycosides.

### [Background Art]

Polyketides are a class of attractive natural products having various biological effects, and are widely applied to foods, cosmetics, drugs, and the like in daily life. Enzymes that biosynthesize polyketides are collectively referred to as polyketide biosynthesis enzymes (polyketide synthases (PKSs)), which are classified into three types, namely types I, II, and III depending on biosynthesis mechanisms. Thereamong, macrolide-based polyketides are produced through type I PKS, and aromatic polyketides are mainly produced through types II and III.

For materials having medicinal efficacy, such as drugs or nutritional supplements, glycosides with much improved stability, resistance to hydrolysis, bioavailability, etc. are generally preferred over non-glycosides through formation of glycosidic bonds. In particular, stable C-glycosidic bonds are chemically more stable than O-glycosidic bonds. However, although a few cases of O-glycosylation of natural products have been reported in *Escherichia coli* (Chen, D.; Chen, R. ; Xie, K.; Duan, Y.; Dai, J., Production of acetophenone C-glucosides using an engineered C-glycosyltransferase in Escherichia coli. Tetrahedron Lett. 2018, 59 (19), 1875-1878), almost no cases of C-glycosylation have been reported. There have been many reports about O-glycosylation rather than C-glycosylation in nature as well as in *E. coli.*

Typical C-glycoside natural products include carminic acid, aloesin, and the like.

Carminic acid is a widely used red dye and is used in food, cosmetics, and pharmaceuticals. Carminic acid is extracted directly from scale insects such as cochineal *Dactylopius coccus,* and is added to foods such as ketchup, strawberry milk, candy, and the like, and to cosmetics such as eye shadow, nail polish, lipstick, and the like. However, the cochineal is slow-growing and grows only in limited areas (it may only grow in hot and dry regions), making it difficult to increase production capacity easily, which limits commercial production. In particular, the extraction process is also very inefficient; for example, 70,000 female cochineal insects are required to produce one pound of carminic acid. Under these circumstances, it is necessary to develop a more sustainable method for producing carminic acid.

Aloesin is extracted from *Aloe vera* and is widely used as a whitening agent in the cosmetic industry due to anti-tyrosinase effects and anti-melanin production effects thereof. Moreover, since aloesin shows anti-inflammatory and anti-radical effects, it may be used as a main ingredient of various drugs or cosmetics. However, the amount of aloesin extracted from aloe plants is extremely small, so it is necessary to develop a more efficient and sustainable bio-based production method.

As described above, the demand for C-glycoside natural products is very high, whereas the supply thereof is insufficient, but development of methods capable of effectively producing the same has not yet been made. In particular, even when the compound is to be produced through a biological process, enzymes therefor are not well known, or efficient production from a microbial cell factory is impossible owing to low enzyme conversion efficiency.

Against this technical background, the present inventors have made great efforts to develop C-glycosyltransferase having excellent C-glycosylation capability, and thus developed a C-glycosyltransferase variant that exhibits excellent C-glycosylation capability through amino acid substitution and ascertained that, in a recombinant microorganism into which the C-glycosyltransferase variant gene is introduced, the ability of the C-glycosyltransferase variant to produce glycosides is very high for type I, type II, and type III polyketides, non-ribosomal peptides, phenylpropanoids, and aromatic natural products, thus culminating in the present invention.

The above information described in the background section is only for improving the understanding of the background of the present invention, and it does not include information forming the prior art known to those of ordinary skill in the art to which the present invention pertains.

### [Citation List]

### [Non-Patent Literature]

(Non-Patent Document 1) Chen, D.; Chen, R. ; Xie, K. ; Duan, Y.; Dai, J., Production of acetophenone C-glucosides using an engineered C-glycosyltransferase in Escherichia coli. Tetrahedron Lett. 2018, 59 (19), 1875-1878

### [Disclosure]

It is an object of the present invention to provide a novel C-glycosyltransferase variant and the use thereof.

In order to accomplish the above object,
the present invention provides a C-glycosyltransferase variant including a mutation in at least one amino acid selected from the group consisting of F17, V93, V132, Y193, L164, and R322 in the C-glycosyltransferase represented by SEQ ID NO: 1.

In addition, the present invention provides a nucleic acid encoding the C-glycosyltransferase variant.

In addition, the present invention provides a recombinant microorganism into which the nucleic acid is introduced.

In addition, the present invention provides a method of producing a polyketide glycoside and/or a phenylpropanoid glycoside, including:
(a) producing a polyketide glycoside and/or phenylpropanoid glycoside by culturing the recombinant microorganism of the present invention; and
(b) recovering the produced polyketide glycoside and/or phenylpropanoid glycoside.

In addition, the present invention provides a method of producing a polyketide glycoside and/or a phenylpropanoid glycoside, including:
(a) producing a polyketide glycoside and/or phenylpropanoid glycoside by reacting the C-glycosyltransferase variant of the present invention or a microorganism expressing the C-glycosyltransferase variant with a polyketide and/or phenylpropanoid; and
(b) recovering the produced polyketide glycoside and/or phenylpropanoid glycoside.

### [Description of Drawings]

FIG. 1 shows the carminic acid production pathway;
FIG. 2 shows flavokermesic acid production when different metabolic engineering strategies are introduced, in which FK was produced in a higher concentration when using type II polyketide synthase (AntDEFBG from *P. luminescens*) and ZhuIJ than when using type III polyketide synthase (AaPKS5 from *Aloe arborescens*) and ZhuIJ;
FIG. 3 shows changes in kermesic acid production depending on the introduction of DnrF;
FIG. 4 shows C-glycosyltransferase candidates for production of dcII and original enzymatic reactions of the candidate enzymes;
FIG. 5 shows results of comparison of the dcII production capacities of nine enzyme candidates;
FIG. 6 shows results of homology modeling and docking simulation for increasing KA and dcII production: (a) KA production capacity of variants selected through simulation for DnrF, (b) protein structure simulation results for the most effective DnrF variant (P217K), (c) dcII production capacity of variants selected through simulation for GtCGT, and (d) protein structure for the most effective GtCGT variant (V93Q/Y193F);

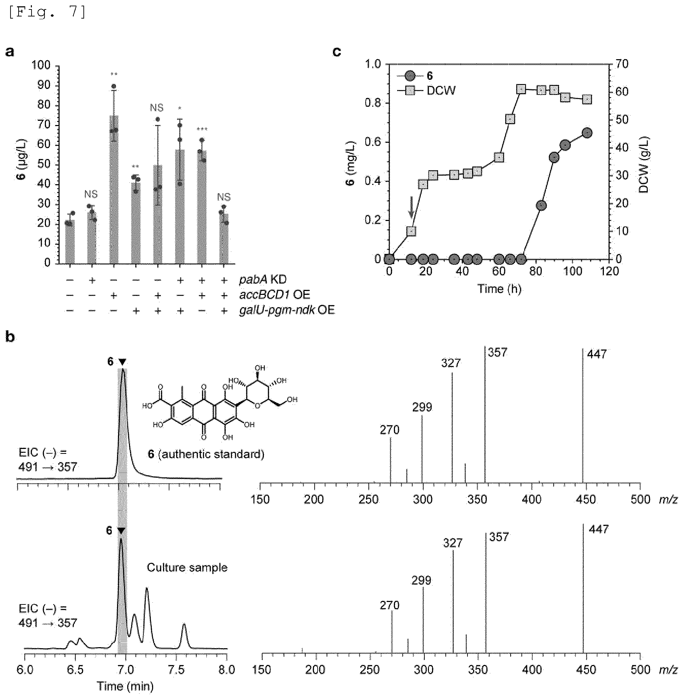

FIG. 7 shows production of carminic acid from glucose: (a) carminic acid production under different conditions, (b) analysis of carminic acid by LC-MS/MS, the upper data showing results of analyzing commercially available carminic acid, the lower data showing results of analyzing a sample containing carminic acid produced in *E. coli* from glucose, left graphs showing extracted ion chromatogram (EIC), and right graphs showing MS/MS fragmentation pattern, and (c) a graph of fed-batch fermentation of the final strain, the red arrow representing the time point of gene expression onset through IPTG, and DCW representing dry cell weight;
FIG. 8 shows the aloesin production pathway;
FIG. 9 shows production of aloesin via *E. coli:* (a) test results of constructing another plasmid containing RpALS for increased production of aloesone, (b) test results of GtCGT and variants thereof for production of aloesin, and (c) analysis of aloesin through LC-MS/MS, the upper data showing results of analyzing commercially available aloesin, the lower data showing results of analyzing a sample containing aloesin produced in *E. coli* from glucose, left graphs showing extracted ion chromatogram (EIC), and right graphs showing MS/MS fragmentation pattern;
FIG. 10 shows test results of additional variants of GtCGT for increasing aloesin production, in which additional variants were predicted by analyzing the structural model of the GtCGT variant (V93Q/Y193F);
FIG. 11 shows test results of additional variants of GtCGT for increasing aloesin production, in which additional variants were predicted by performing docking simulation based on the GtCGT variant (V93Q/Y193F);
FIG. 12 shows the production (represented as %conversion) of various phenylpropanoid C-glucosides by the GtCGT variant (V93Q/Y193F); and
FIG. 13 shows a Lineweaver-Burk plot for calculating K_{M} and Vₘₐₓ values of GtCGT and the GtCGT variant (V93Q/Y193F).

### [Mode for Invention]

Unless otherwise defined, all technical and scientific terms used herein have the same meanings as typically understood by those skilled in the art to which the present invention belongs. In general, the nomenclature used herein is well known in the art and is typical.

In the present invention, in order to discover C-glycosyltransferase variants having very high ability to form glycosidic bonds compared to wild-type enzymes, protein structures were predicted, and variant candidates with increased activity were obtained through protein structure analysis and computer simulation, among which effective variants with increased ability to bind to a substrate and capable of enhancing glucosylation reaction could be selected.

Accordingly, an aspect of the present invention pertains to a C-glycosyltransferase variant having increased C-glycosylation capability.

In the present invention, C-glycosyltransferase, which is the template (or wild type) of the variant according to the present invention, is an enzyme that induces C-glycosylation by forming a C-glycosidic bond to carbon of a substrate (e.g. a compound, protein, etc.).

In the present invention, the C-glycosyltransferase is represented by SEQ ID NO: 1, but is not limited thereto, and is to be understood as including a protein in which an amino acid residue is subjected to conserved substitution at a certain amino acid residue position.

In the present invention, "conserved substitution" is modification of C-glycosyltransferase including substitution of at least one amino acid with an amino acid having similar biochemical properties that do not cause loss of biological or biochemical functions of C-glycosyltransferase or variants thereof.

In the present invention, "conserved amino acid substitution" is substitution in which an amino acid residue is replaced with an amino acid residue having a similar side chain. Classes of amino acid residues having similar side chains are defined in the art and are well known. These classes include amino acids having basic side chains (e.g. lysine, arginine, histidine), amino acids having acidic side chains (e.g. aspartic acid, glutamic acid), uncharged amino acids having polar side chains (e.g. glycine, asparagine, glutamine, serine, threonine, tyrosine, cysteine), amino acids having non-polar side chains (e.g. alanine, valine, leucine, isoleucine, proline, phenylalanine, methionine, tryptophan), amino acids having beta-branched side chains (e.g. threonine, valine, isoleucine), and amino acids having aromatic side chains (e.g. tyrosine, phenylalanine, tryptophan, histidine).

Therefore, the C-glycosyltransferase, which is the template of the variant of the present invention, is to be understood to include all of C-glycosyltransferase, recombinant C-glycosyltransferase, and fragments thereof, not only including SEQ ID NO: 1 but also having substantially the same function and/or effect and having amino acid sequence homology of 400 or more, 500 or more, 600 or more, 700 or more, preferably 80% or more or 85% or more, more preferably 90% or more or 950 or more, most preferably 990 or more.

As used herein, the term "fragment" refers to a part resulting from cleaving a parent protein, and may be obtained through cleavage of the C' -terminus and/or the N'-terminus. The fragment of the present invention is a fragment having substantially the same function and/or effect as the deglycosylated C-glycosyltransferase of the present invention. For example, the fragment may include a fragment in which a signal sequence is cleaved from a full-length protein.

In the present invention, the C-glycosyltransferase may be derived from other strains or organisms, in addition to GtUF6CGT from *Gentiana triflora* represented by SEQ ID NO: 1. Examples thereof may include, but are not limited to, IroB (EnCGT) from *E. coli* Nissle, UGT708A6 (ZmCGT) from *Zea mays* which acts as a dual C/O-glycosyltransferase, UGT708C2 (FeCGT) from *Fagopyrum esculentum,* MiCGT from *Mangifera indica,* OsCGT from *Oryza sativa,* UGT708D1 (GmCGT) from *Glycine max,* GtUF6CGT1 (GtCGT) from *Gentiana triflora,* and AvCGT from *Aloe vera,* preferably GtUF6CGT1 (GtCGT) from *Gentiana triflora* or UGT708A6 (ZmCGT) from *Zea mays* which acts as a dual C/O-glycosyltransferase.

In an embodiment of the present invention, it was confirmed that, when a variant is produced by substituting a portion of amino acids of wild-type C-glycosyltransferase, it exhibits very high ability to induce C-glycosylation and also that, when the C-glycosyltransferase is introduced into a recombinant strain for polyketide synthesis, C-glycosylated polyketide may be produced in a high yield.

In the present invention, the C-glycosyltransferase variant may include a mutation in at least one amino acid selected from the group consisting of F17, V93, V132, Y193, L164, and R322 in the C-glycosyltransferase represented by SEQ ID NO: 1, and preferably includes a mutation in amino acids V93 and/or Y193.

In the present invention, the C-glycosyltransferase variant may include a mutation in one or more other amino acids, in addition to at least one amino acid selected from the group consisting of F17, V93, V132, Y193, L164, and R322 in the C-glycosyltransferase represented by SEQ ID NO: 1.

In the present invention, the C-glycosyltransferase variant may further include a mutation in at least one amino acid selected from the group consisting of F17, V405, P107, L208, L164, P45, I305, L316, F401, Y94, N57, Y187, C16, P319, F167, V132, N206, R406, Q386, V129, L125, L194, I95, S215, L184, Y158, L29, L27, F202, H159, S370, H365, V329, M301, V315, V190, C366, W80, L58, Q210, F312, D61, I207, L363, P196, L106, V93, A394, W314, S155, P88, D99, Y284, E189, G49, H328, E399, T392, F387, A44, P199, E46, R28, V285, I124, R419, L306, Y157, Y200, E373, P191, L214, S376, V15, E332, E51, I417, L98, I323, H161, T383, P127, E309, N84, L313, Q104, T371, N213, G79, L330, N307, K105, L128, A152, , I18, N59, W147, S86, L293, E296, S377, L185, K216, F89, S286, F396, F211, Y303, D223, R415, N96, V22, S153, F154, D192, Y193, H195, P201, Y292, and R322 in the C-glycosyltransferase represented by SEQ ID NO: 1.

In the present invention, the C-glycosyltransferase variant may further include a mutation in at least one amino acid selected from the group consisting of I18, Q20, T50, I95, V290, I323, V22, L29, E46, V48, E51, A55, S86, D99, R103, C151, L184, L194, E332, and P385 in the C-glycosyltransferase represented by SEQ ID NO: 1.

In the present invention, the C-glycosyltransferase variant preferably further includes a mutation in at least one amino acid selected from the group consisting of I323, T50, I18, I95, Q20, P385, L194, and V48 in the C-glycosyltransferase represented by SEQ ID NO: 1.

As used herein, the term "variant" is used as a concept encompassing all of not only mutation of some amino acid residues, preferably substitution, deletion, and/or insertion of amino acid residues, more preferably substitution of amino acid residues, in the amino acid sequence of a reference sequence (e.g. a wild-type C-glycosyltransferase sequence, SEQ ID NO: 1), but also deletion of some amino acid residues at the N-terminus or C-terminus, along with substitution, deletion, and/or insertion of such amino acid residues. In an embodiment of the present invention, the variant was produced by substituting some amino acids of SEQ ID NO: 1, but the present invention is not limited thereto.

In the present invention, the 'mutation' may be amino acid substitution.

In the present invention, the C-glycosyltransferase variant may include substitution of at least one amino acid selected from the group consisting of F17G, V93Q, V132A, Y193F, L164G, and R322D in the C-glycosyltransferase represented by SEQ ID NO: 1, more preferably substitution of amino acids V93Q and/or Y193F, most preferably substitution of amino acids V93Q and Y193F.

In the present invention, the C-glycosyltransferase variant may further include substitution of at least one different amino acid, in addition to the substitution of at least one amino acid selected from the group consisting of F17G, V93Q, V132A, Y193F, L164G, and R322D in the C-glycosyltransferase represented by SEQ ID NO: 1.

In the present invention, the C-glycosyltransferase variant may further include substitution of at least one different amino acid, in addition to the substitution of amino acids V93Q and Y193F in the C-glycosyltransferase represented by SEQ ID NO: 1.

In the present invention, the substitution of the different amino acid that may be further included in the C-glycosyltransferase variant may be substitution of at least one amino acid selected from the group consisting of F17G, V405M, P107G, L208G, L164G, P45G, I305A, L316G, F401H, Y94G, N57G, Y187A, C16G, P319G, F167G, V132A, N206E, R406G, Q386H, V129A, L125V, L194A, I95G, S215D, L184G, Y158T, L29A, L27A, F202S, H159G, S370A, H365G, V329T, M301W, V315A, V190A, C366G, W80Y, L58E, Q210G, F312G, D61G, I207P, L363G, P196G, L106G, V93G, A394G, W314C, S155A, P88D, D99G, Y284H, E189A, G49TH328G, E399D, T392A, F387T, A44G, P199E, E46G, R28G, V285I, I124T, R419A, L306M, Y157T, Y200L, E373A, P201G, P191G, L214A, S376G, V15G, E332P, E51C, I417L, L98G, I323A, H161G, T383C, P127A, E309N, N84S, L313T, Q104D, T371A, N213L, G79S, L330G, N307A, K105G, L128D, A152G, S153G, I18A, N59V, W147F, S86V, L293V, E296D, S377A, L185V, K216R, F89A, S286C, F396L, F211G, Y303A, D223G, R415L, N96A, V22H, V93Q, V93L, S153C, F154L, D192S, Y193F, H195Y, H195L, P201T, Y292H, Y292F, R322D, and R322A in the C-glycosyltransferase represented by SEQ ID NO: 1.

In the present invention, the substitution of the different amino acid that may be further included may be substitution of at least one amino acid selected from the group consisting of I18P, Q20M, T50N, T50Q, T50K, T50R, T50V, I95M, I95T, V290G, V290A, I323S, I323A, I95L, V22A, L29A, E46G, V48G, E51C, A55S, S86V, D99G, R103V, C151G, L184G, L194A, E332P, I18A, and P385A in the C-glycosyltransferase represented by SEQ ID NO: 1.

In the present invention, the substitution of the different amino acid that may be further included is preferably substitution of at least one amino acid selected from the group consisting of I323S, T50R, T50V, I18P, I95T, Q20M, I323A, P385A, L194A, and V48G in the C-glycosyltransferase represented by SEQ ID NO: 1.

In an embodiment of the present invention, it was confirmed that the C-glycosyltransferase variant shows vastly superior C-glycosylation when including:
i) substitution of amino acids V93Q and Y193F in the C-glycosyltransferase represented by SEQ ID NO: 1;
ii) substitution of amino acids V93Q, Y193F, and I323S in the C-glycosyltransferase represented by SEQ ID NO: 1; or
iii) substitution of amino acids V93Q, Y193F, and P385A in the C-glycosyltransferase represented by SEQ ID NO: 1, but the present invention is not limited thereto.

The position of the amino acid residue in the amino acid mutation may be accurately numbered using the amino acid sequence represented by SEQ ID NO: 1 as a reference. Here, "residue Xn" indicates a residue X at position n in the amino acid sequence represented by SEQ ID NO: 1, in which n is a positive integer and X is an abbreviation for any amino acid residue. For example, "residue V93" indicates an amino acid residue V at position 93 in the amino acid sequence represented by SEQ ID NO: 1.

In the present invention, "amino acid mutation" may be "amino acid substitution Xn", and for instance, refers to amino acid substitution occurring in an amino acid residue X at position n in the amino acid sequence represented by SEQ ID NO: 1, in which n is a positive integer and X is an abbreviation for any amino acid residue. For example, "amino acid substitution V93" indicates amino acid substitution occurring in an amino acid residue V at position 93 in the amino acid sequence represented by SEQ ID NO: 1.

In the present invention, when a C-glycosyltransferase having an amino acid sequence other than the C-glycosyltransferase of SEQ ID NO: 1 used as the reference sequence is used as a reference sequence, an amino acid residue corresponding to the specific amino acid residue set forth with reference to SEQ ID NO: 1 may generally be obtained by alignment of amino acid sequences under optimized conditions. The sequence alignment may be performed by means understood by those skilled in the art, for example using BLAST, BLAST-2, ALIGN, NEEDLE or MegAlign (DNASTAR) software and the like. Appropriate parameters for use in alignment, including any algorithms needed to achieve optimal alignment over the full-length sequences being compared, may be determined by those skilled in the art.

The amino acid substitution of the present invention may be non-conserved substitution. Such non-conserved substitution may include altering an amino acid residue of a target protein or polypeptide in a non-conservative manner, such as replacement of an amino acid residue having a certain side chain size or a certain property (e.g. hydrophilicity) with an amino acid residue having a different side chain size or a different property (e.g. hydrophobicity).

Also, the amino acid substitution may be conserved substitution. Such conserved substitution may include altering an amino acid residue of a target protein or polypeptide in a conservative manner, such as replacement of an amino acid residue having a certain side chain size or a certain property (e.g. hydrophilicity) with an amino acid residue having the same or similar side chain size or the same or similar property (e.g. hydrophilicity). Conserved substitution usually does not greatly affect the structure or function of the produced protein. In the present application, amino acid sequence variants that are mutations in fusion proteins, fragments thereof, or variants thereof in which one or more amino acids are substituted may include conserved amino acid substitution that does not greatly change the structure or function of the protein.

For example, mutual substitution between amino acids in each of the following groups may be regarded as conserved substitution in the present application:
a group of amino acids having non-polar side chains: alanine, valine, leucine, isoleucine, proline, phenylalanine, tryptophan, and methionine;
a group of uncharged amino acids having polar side chains: glycine, serine, threonine, cysteine, tyrosine, asparagine, and glutamine;
a group of negatively charged amino acids having polar side chains: aspartic acid and glutamic acid;
a group of positively charged basic amino acids: lysine, arginine, and histidine; and
a group of amino acids having phenyl: phenylalanine, tryptophan, and tyrosine.

The proteins, polypeptides, and/or amino acid sequences encompassed by the present invention may also be understood to include at least the following ranges: variants or homologues having the same or similar functions as the proteins or polypeptides.

In the present invention, the variant may be a protein or polypeptide produced by substitution, deletion, or addition of at least one amino acid as compared with the amino acid sequence of wild-type C-glycosyltransferase. For example, the functional variant may include proteins or polypeptides with amino acid changes by substitution, deletion, and/or insertion of at least one amino acid, for example 1-30, 1-20, or 1-10 amino acids, or alternatively 1, 2, 3, 4, or 5 amino acids. The functional variant may substantially retain biological properties of the protein or polypeptide before changes (e.g. substitution, deletion, or addition). For example, the functional variant may retain at least 60%, 70%, 80%, 90%, or 100% of the biological activity of the protein or polypeptide before changes.

In the present invention, the homologue may be a protein or polypeptide having sequence homology of at least about 80% (e.g. at least about 85%, about 90%, about 910, about 92%, about 93%, about 94%, about 95%, about 96%, about 97%, about 98%, or about 990) with the amino acid sequence of the protein and/or the polypeptide.

In the present invention, the homology generally indicates similarity, analogousness, or association between two or more sequences. The "percentage of sequence homology" may be calculated by comparing two aligned sequences in a comparison window that determines the number of positions where identical nucleic acid bases (e.g. A, T, C, G, I) or identical amino acid residues (e.g. Ala, Pro, Ser, Thr, Gly, Val, Leu, Ile, Phe, Tyr, Trp, Lys, Arg, His, Asp, Glu, Asn, Gln, Cys, and Met) are present, and the percentage of sequence homology may be obtained by dividing the number of matching positions by the total number of positions and then multiplying the result by 100 in order to provide the number of matching positions in the comparison window (i.e. window size). Alignment for determining the percentage of sequence homology may be performed in a variety of ways known in the art, for example using publicly available computer software such as BLAST, BLAST-2, ALIGN, or MegAlign (DNASTAR) software. Appropriate parameters for aligning sequences, including any algorithms necessary to achieve maximal alignment within the full-length sequences being compared or within the target sequence region may be determined by those skilled in the art. The homology may also be determined by the following method: FASTA and BLAST. The FASTA algorithm is described, for example, in W. R. Pearson and D. J. Lipman's "Improved Tool for Biological Sequence Comparison", Proc. Natl. Acad. Sci., 85: 2444-2448, 1988; and D, J. Lipman and W. R. Pearson's "Fast and Sensitive Protein Similarity Search", Science, 227:1435-1441, 1989, and a description of the BLAST algorithm is provided by S. Altschul, W. Gish, W. Miller, E. W. Myers and D. Lipman, "A Basic Local Alignment Search Tool", Journal of Molecular Biology, 215: 403-410, 1990.

In the present invention, the C-glycosyltransferase variant may be characterized by enhanced glycosylation reaction of substrate carbon compared to the wild type.

In the present invention, the mutated amino acid is located at the active site of the enzyme, and through amino acid mutation, the ability of the variant to bind to a substrate may be increased by 10% or more, preferably 200 or more, more preferably 500 or more compared to the wild type.

In an embodiment of the present invention, it was confirmed that the C-glycosyltransferase variant of the present invention highly facilitates C-glycosylation of various polyketide-based compounds (flavokermesic acid, kermesic acid, aloesone) or phenylpropanoid-based compounds (naringenin, apigenin, or luteolin) serving as a substrate, regardless of the type of substrate, compared to the wild type. Therefore, the C-glycosyltransferase variant of the present invention may be used for C-glycosylation of various compounds or proteins serving as the substrate. For example, the C-glycosyltransferase variant of the present invention may be used for C-glycosylation of the polyketide-based compound or the phenylpropanoid-based compound, but the present invention is not limited thereto.

In the present invention, polyketide- or phenylpropanoid-based compounds may be used without limitation as the substrate, and as confirmed in an embodiment, preferable examples of the substrate include, but are not limited to, flavokermesic acid, kermesic acid, aloesone, naringenin, apigenin, and luteolin.

The substrate is preferably flavokermesic acid or kermesic acid, and the variant enables glycosylation at carbon number 2 of flavokermesic acid, but the present invention is not limited thereto.

The substrate is preferably aloesone, and the variant enables glycosylation at carbon number 8 of aloesone, but the present invention is not limited thereto.

Another aspect of the present invention pertains to a nucleic acid encoding the C-glycosyltransferase variant.

Still another aspect of the invention pertains to a vector including the nucleic acid.

Yet another aspect of the present invention pertains to a recombinant microorganism into which the nucleic acid is introduced.

In the present invention, the recombinant microorganism may be configured such that the nucleic acid is introduced in the form of a plasmid into a host microorganism or is inserted into the genome.

In the present invention, the recombinant microorganism may be used for production of a polyketide glycoside and/or a phenylpropanoid glycoside, but the present invention is not limited thereto.

In the present invention, the recombinant microorganism may have the ability to produce a polyketide and/or a phenylpropanoid as a substrate of the C-glycosyltransferase of the present invention, and the polyketide and/or the phenylpropanoid may be glycosylated by C-glycosyltransferase expressed by the recombinant microorganism of the present invention and thus converted into a polyketide glycoside and/or a phenylpropanoid glycoside.

In the present invention, the polyketide may be selected from the group consisting of, but is not limited to:
type I polyketide selected from the group consisting of rapamycin, lovastatin, erythromycin, rifamycin, avermectin, geldanamycin, ivermectin, calicheamicin, epothilone, triacetic acid lactone, and 6-methylsalicylic acid;
type II polyketide selected from the group consisting of actinorhodin, doxorubicin, daunorubicin, oxytetracycline, SEK4, SEK4b, SEK34, SEK15, SEK26, FK506, DMAC, aklavinone, aklanonic acid, epsilon-rhodomycinone, doxycycline, anthramycin, tetracenomycin, carminic acid, and frenolicin; and
type III polyketide selected from the group consisting of aloesin, aloenin, barbaloin, 5,7-dihydroxy-2-methylchromone, and aloesone.
In the present invention, the phenylpropanoid may be selected from the group consisting of, but is not limited to:
non-ribosomal peptides, including actinomycin, bacitracin, daptomycin, vancomycin, teixobactin, tyrocidine, gramicidin, zwittermicin A, bleomycin, ciclosporin, pyoverdine, enterobactin, myxochelin A, indigoidine, and cyanophycin, pinocembrin, dihydrokaempferol, eriodictyol, dihydroquercetin, coniferyl alcohol, silybin, isosilybin, silychristin, silinide, 2,3-dehydrosilybin, silydianin, daidzein, genistein, apigenin, luteolin, kaempferol, quercetin, catechin, pelargonidin, cyanidin, afzelechin, myricetin, fisetin, galangin, hesperetin, tangeritin, delphinidin, epicatechin, chrysin, resveratrol, and naringenin.

In the present invention, the host microorganism may have the ability to produce a precursor of the polyketide glycoside and/or phenylpropanoid glycoside of interest.

In the present invention, the precursor of the polyketide glycoside and/or phenylpropanoid glycoside may be a polyketide and/or phenylpropanoid, preferably a non-glycosylated polyketide and/or phenylpropanoid.

In the present invention, the host microorganism is able to innately produce a precursor of the polyketide glycoside and/or phenylpropanoid glycoside, or may be a recombinant microorganism prepared to produce a precursor of the polyketide glycoside and/or phenylpropanoid glycoside through genetic manipulation.

In the present invention, the recombinant microorganism may be characterized by enhanced production of nucleotides, preferably NTP-sugar, in order to increase glycoside conversion efficiency by the introduced C-glycosyltransferase. For example, the recombinant microorganism of the present invention may be additionally characterized by enhanced expression of a gene encoding UTP-glucose-1-phosphate uridyltransferase, phosphoglucomutase, and/or nucleoside-diphosphate kinase, but the present invention is not limited thereto.

In the present invention, the UTP-glucose-1-phosphate uridyltransferase, phosphoglucomutase, and/or nucleoside-diphosphate kinase may be derived from *E. coli,* but the present invention is not limited thereto, and expression of a gene involved in the production of NTP-sugar may be enhanced depending on the host strain.

In an embodiment of the present invention, flavokermesic acid, kermesic acid, aloesone, naringenin, apigenin, or luteolin was used as the precursor of the polyketide glycoside and/or phenylpropanoid glycoside, but the present invention is not limited thereto, and various precursors of the polyketide glycoside and/or phenylpropanoid glycoside described above are well known in the art, and may be easily selected therefrom.

As used herein, the term "nucleic acid" refers to a nucleotide, deoxyribonucleotide or ribonucleotide, or analogues thereof of any length, in an isolated form, generally isolated from the natural environment or artificially synthesized. The nucleic acid of the present invention may be isolated. For example, nucleic acid may be produced or synthesized in the following manner: (i) *in-vitro* amplification, such as polymerase chain reaction (PCR) amplification, (ii) clonal recombination, (iii) purification, such as fractionation by restriction enzyme digestion and gel electrophoresis, or (iv) synthesis, such as chemical synthesis. In some embodiments, the isolated nucleic acid is a nucleic acid molecule produced by recombinant DNA technology. In the present invention, a nucleic acid encoding the variant may be produced by various methods known in the art. Such methods include, but are not limited to, restriction fragment processing or overlap extension PCR using synthetic oligonucleotides. The production method and principle are described in Sambrook et al., Molecular Cloning, A Laboratory Manual, Cold Spring Harbor Laboratory Press, Cold Spring Harbor, NY, 1989; and Auube et al. Current Protocols in Molecular Biology, Greene Publishing and Wiley-Interscience, New York NY, 1993.

As used herein, the term "plasmid" may be used interchangeably with vector, and generally refers to a nucleic acid molecule capable of self-replication in a host cell or microorganism by delivering an inserted nucleic acid to the host cell (including a host microorganism). Examples of the vector may include vectors primarily used for inserting DNA or RNA into cells, vectors primarily used for DNA or RNA replication, and vectors primarily used for transcriptional and/or translational expression of DNA or RNA. Examples of the vector may also include vectors with multiple functions. The vector may be a polynucleotide that may be transcribed and translated into a polypeptide when introduced into a suitable host cell. Generally, the vector is able to produce a desired expression product by culturing a suitable host cell containing the vector. In the present invention, the vector may include at least one of the nucleic acids. For example, the vector may include all nucleic acid molecules necessary to encode the variant. As such, only one vector is needed to obtain the fusion protein of the present application. In some embodiments, the vector may include a nucleic acid molecule encoding a portion of the variant. Alternatively, the vector may include a nucleic acid molecule, for example, for regulating gene expression in the recombinant microorganism. Here, two or more different vectors may be required to obtain the recombinant microorganism of the present invention.

In addition, the vector may include other genes, such as marker genes, which select the vector in an appropriate host cell and under appropriate conditions. Also, the vector may include an expression control element to properly express the coding region in a suitable host. These control elements are well known to those skilled in the art. For example, they may include promoters, ribosome binding sites, enhancers, and other control elements that regulate gene transcription or mRNA translation. In some embodiments, the expression control sequence is a regulatory element. The specific structure of the expression control sequence may vary depending on the species or the function of cell type, but generally includes 5' and 3' non-translated sequences as well as 5' non-transcribed sequences involved in transcription and translation initiation, such as TATA boxes, capped sequences, CAAT sequences, etc. For example, the 5' non-transcribed expression control sequence may include a promoter region, and the promoter region may include a promoter sequence for transcriptional control of a functionally linked nucleic acid. In the present invention, the vector may be selected from the group consisting of pET-30a-c (+), pET-22b (+), pCDFDuet-1, pACYCDuet-1, pRSFDuet-1, pBBR1MCS, pSC101, pTac15K, pTrc99A, pCOLADuet-1, and pBR322, but is not limited thereto, and those skilled in the art will be able to appropriately select and use vectors commonly used in the art in addition to the vectors described above.

In the present invention, the terms "host cell", "cell", "host microorganism", and "host" may be used interchangeably and generally refer to an individual cell, cell line, microorganism, or cell culture capable of expressing a plasmid or vector including or capable of including the nucleic acid of the present invention, the variant of the present invention, or proteins or polypeptides, expression of which is regulated. The host cell may include progeny of a single host cell. Due to natural, accidental, or deliberate mutation, the progeny cell and the original parental cell are not necessarily completely identical in morphology or genome, so long as they are able to express the target protein or polypeptide of the present invention. The host cell may be obtained by transfecting cells *in vitro* with the vector of the present invention. The host cell is preferably a microorganism, and may be selected from the group consisting of, for example, E. *coli., Rhizobium, Bifidobacterium, Candida, Erwinia, Enterobacter, Pasteurella, Mannheimia, Actinobacillus, Aggregatibacter, Xanthomonas, Vibrio, Azotobacter, Acinetobacter, Ralstonia, Agrobacterium, Rhodobacter, Zymomonas, Bacillus, Staphylococcus, Lactococcus, Streptococcus, Lactobacillus, Clostridium, Corynebacterium, Streptomyces, Bifidobacterium, Cyanobacterium,* and *Cyclobacterium,* but is not limited thereto.

Also, in the present invention, it was confirmed that various polyketide glycosides or phenylpropanoid glycosides may be effectively produced using the recombinant microorganism capable of expressing the C-glycosyltransferase variant.

Therefore, still yet another aspect of the present invention pertains to a recombinant microorganism for producing a polyketide glycoside or a phenylpropanoid glycoside into which the nucleic acid encoding the C-glycosyltransferase variant of the present invention is introduced.

In the present invention, the polyketide glycoside may be type I polyketide glycoside, type II polyketide glycoside, or type III polyketide glycoside.

In the present invention, the polyketide may be selected from the group consisting of, but is not limited to:
type I polyketide selected from the group consisting of rapamycin, lovastatin, erythromycin, rifamycin, avermectin, geldanamycin, ivermectin, calicheamicin, epothilone, triacetic acid lactone, and 6-methylsalicylic acid;
type II polyketide selected from the group consisting of actinorhodin, doxorubicin, daunorubicin, oxytetracycline, SEK4, SEK4b, SEK34, SEK15, SEK26, FK506, DMAC, aklavinone, aklanonic acid, epsilon-rhodomycinone, doxycycline, anthramycin, tetracenomycin, carminic acid, and frenolicin; and
type III polyketide selected from the group consisting of aloesin, aloenin, barbaloin, 5,7-dihydroxy-2-methylchromone, and aloesone.

In the present invention, the recombinant microorganism for producing the polyketide glycoside or phenylpropanoid glycoside is able to produce a precursor of each glycoside. For example, the recombinant microorganism is able to produce a polyketide or phenylpropanoid, which is the precursor of each glycoside.

In the present invention, the recombinant microorganism for producing the polyketide glycoside or phenylpropanoid glycoside is able to produce a polyketide or phenylpropanoid through additional gene introduction. Recombinant microorganisms capable of synthesizing polyketides through gene introduction may be prepared using genes and methods described in the published paper of the present inventors, for example, Yang, D., Kim, W.J., Yoo, S.M., Choi, J.H., Ha, S.H., Lee, M.H., and Lee, S.Y. "Repurposing type III polyketide synthase as a malonyl-CoA biosensor for metabolic engineering in bacteria", Proc. Natl. Acad. Sci. (PNAS), 115 (40) 9835-9844 (https://doi.org/10.1073/pnas.1808567115) (2018.10.2), and Korean Patent No. 10-2187682, but the present invention is not limited thereto. Recombinant microorganisms capable of synthesizing polyketides or phenylpropanoids may be produced by those skilled in the art through introduction into various host microorganisms using various polyketide or phenylpropanoid synthesis pathways described in the art and genes involved therein.

In the present invention, the recombinant microorganism for producing the polyketide glycoside or phenylpropanoid glycoside may be additionally characterized in that polyketide synthase or phenylpropanoid synthetase is introduced thereto.

In the present invention, examples of the polyketide synthase may include, but are not limited to, type I polyketide synthase, type II polyketide synthase, and type III polyketide synthase.

In the present invention, when the recombinant microorganism for producing the polyketide glycoside or phenylpropanoid glycoside does not produce the precursor of each glycoside, the precursor of each glycoside may be added to a culture medium to obtain the polyketide glycoside or phenylpropanoid glycoside.

In the present invention, the recombinant microorganism may be used for production of type I polyketide glycoside.

In the present invention, the recombinant microorganism for producing the type I polyketide glycoside is able to produce a precursor of the type I polyketide glycoside. Examples of the precursor of the type I polyketide glycoside may include, but are not limited to, rapamycin, lovastatin, erythromycin, rifamycin, and the like.

In the present invention, the recombinant microorganism for producing the type I polyketide glycoside is able to produce a precursor of the type I polyketide glycoside through additional gene introduction.

In the present invention, the recombinant microorganism for producing the type I polyketide glycoside may be characterized in that:
(i) a gene encoding type I polyketide synthase is additionally introduced thereto.

In the present invention, the type I polyketide synthase may be easily selected from various protein and gene databases.

Thus, the host microorganism, into which the nucleic acid encoding the C-glycosyltransferase variant of the present invention and the type I polyketide synthase gene are introduced, may have the ability to produce coenzyme A, preferably malonyl-CoA or acetyl-CoA.

Therefore, in the present invention, the recombinant microorganism may be characterized in that production of coenzyme A is enhanced. For example, in the present invention, the recombinant microorganism may be additionally characterized in that (ii) expression of the pabA gene is suppressed or attenuated, but the present invention is not limited thereto, and recombinant microorganisms with enhanced production of coenzyme A may be prepared using various strategies for mass production of coenzyme A known in the art.

In the present invention, the recombinant microorganism may be characterized by enhanced production of nucleotides, preferably NTP-sugar, in order to increase glycoside conversion efficiency by the introduced C-glycosyltransferase. For example, in the present invention, the recombinant microorganism may be additionally characterized in that (iii) expression of a gene encoding UTP-glucose-1-phosphate uridyltransferase, phosphoglucomutase, and/or nucleoside-diphosphate kinase is enhanced, but the present invention is not limited thereto.

In the present invention, the recombinant microorganism may be used for production of type II polyketide glycoside. For example, the type II polyketide glycoside may be carminic acid, but is not limited thereto.

In the present invention, the recombinant microorganism for producing the type II polyketide glycoside is able to produce a precursor of the type II polyketide glycoside. For example, the precursor of the type II polyketide glycoside may be flavokermesic acid or kermesic acid, but is not limited thereto.

In the present invention, the recombinant microorganism for producing the type II polyketide glycoside is able to produce a precursor of the type II polyketide glycoside through additional gene introduction.

In the present invention, the recombinant microorganism for producing the type II polyketide glycoside may be characterized in that (i) a gene encoding type II polyketide synthase is additionally introduced thereto.

In the present invention, the recombinant microorganism for producing the type II polyketide glycoside, preferably carminic acid, may be characterized in that any one or more genes selected from the group consisting of the following, preferably all of the genes are additionally introduced thereto:
(i) a gene encoding type II polyketide synthase;
(ii) a gene encoding 4'-phosphopantetheinyl transferase;
(iii) a gene encoding cyclase;
(iv) a gene encoding acetyl-CoA carboxylase; and
(v) a gene encoding aklavinone 12-hydroxylase.

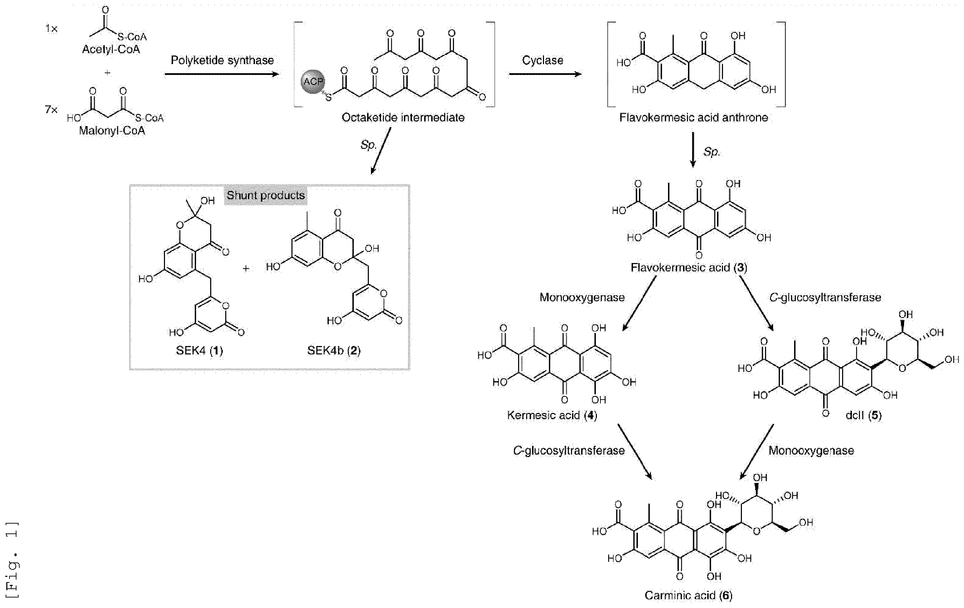

As shown in FIG. 1, for the type II polyketide, which is a substrate of the C-glycosyltransferase of the present invention, for example, coenzyme A (CoA) such as malonyl-CoA or acetyl-CoA may be converted into type II polyketide, which is a substrate of the C-glycosyltransferase of the present invention, by the enzyme encoded by the introduced gene. Thus, the host microorganism into which the nucleic acid encoding the C-glycosyltransferase variant and the type II polyketide synthase gene or the gene of (i) to (v) above are introduced may have the ability to produce coenzyme A, preferably malonyl-CoA or acetyl-CoA.

In an embodiment of the present invention, it was confirmed that coenzyme A accumulates through suppression or attenuation of expression of the pabA gene, thereby improving synthesis of polyketide, which is a precursor of the C-glycosyltransferase of the present invention.

Therefore, in the present invention, the recombinant microorganism may be characterized in that production of coenzyme A is enhanced. For example, in the present invention, the recombinant microorganism may be additionally characterized in that (ii) expression of the *pabA* gene is suppressed or attenuated, but the present invention is not limited thereto, and recombinant microorganisms with enhanced production of coenzyme A may be prepared using various strategies for mass production of coenzyme A known in the art.

In the present invention, the recombinant microorganism may be characterized by enhanced production of nucleotides, preferably NTP-sugar, in order to increase glycoside conversion efficiency by the introduced C-glycosyltransferase. For example, in the present invention, the recombinant microorganism may be additionally characterized in that (iii) expression of a gene encoding UTP-glucose-1-phosphate uridyltransferase, phosphoglucomutase, and/or nucleoside-diphosphate kinase is enhanced, but the present invention is not limited thereto.

In the present invention, the UTP-glucose-1-phosphate uridyltransferase, phosphoglucomutase, and/or nucleoside-diphosphate kinase may be derived from *E. coli,* but the present invention is not limited thereto, and expression of a gene involved in the production of NTP-sugar may be enhanced depending on the host strain.

In the present invention, the gene encoding the type II polyketide synthase may be at least one selected from the group consisting of *antD* (ketosynthase), *antE* (chain-length factor), *antF* (ACP), *antB* (phosphopantetheinyl transferase), and *antG* (malonyl-CoA:ACP malonyl transferase), or combinations thereof, but is not limited thereto.

In the present invention, the aklavinone 12-hydroxylase may include a mutation from proline to lysine (P217K) at amino acid position 217 in the amino acid sequence represented by SEQ ID NO: 2, but is not limited thereto.

In the present invention, the type II polyketide synthase may be derived from *P. luminescens;*
the 4'-phosphopantetheinyl transferase may be derived from *Bacillus subtilis* or *P. luminescens;*
the cyclase may be derived from *Streptomyces sp.;*
the acetyl-CoA carboxylase may be derived from *Corynebacterium glutamicum;* and/or
the aklavinone 12-hydroxylase may be derived from *Streptomyces peucetius,* but the present invention is not limited thereto.

In the present invention, the recombinant microorganism may be used for production of type III polyketide glycoside. For example, in the present invention, the type III polyketide glycoside may be aloesin, but is not limited thereto.

In the present invention, the recombinant microorganism for producing the type III polyketide glycoside is able to produce a precursor of the type III polyketide glycoside. For example, the precursor of the type III polyketide glycoside may be aloesone, but is not limited thereto.

In the present invention, the recombinant microorganism for producing the type III polyketide glycoside is able to produce a precursor of the type III polyketide glycoside through additional gene introduction.

In the present invention, the recombinant microorganism for producing the type III polyketide glycoside may be characterized in that:
(i) a gene encoding type III polyketide synthase is introduced thereto. For example, the type III polyketide synthase may be aloesone synthase, but is not limited thereto.

In the present invention, the aloesone synthase may be derived from *R. palmatum,* but the present invention is not limited thereto.

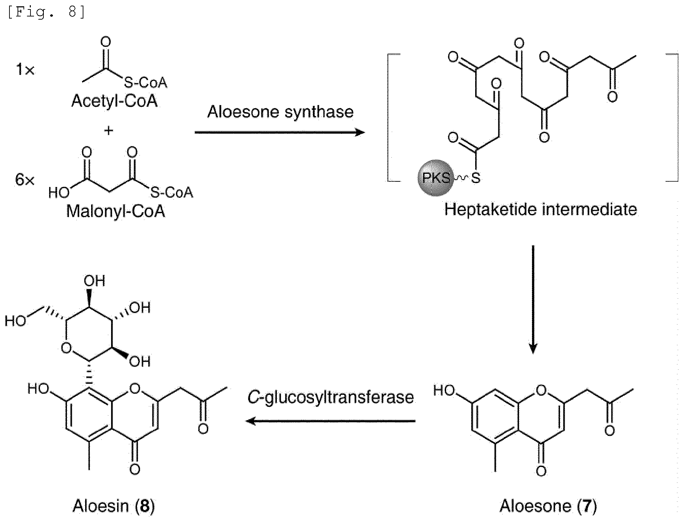

As shown in FIG. 8, for the type III polyketide (e.g. aloesone), which is a substrate of the C-glycosyltransferase of the present invention, for example, coenzyme A (CoA) such as malonyl-CoA or acetyl-CoA may be converted into type III polyketide, which is a substrate of the C-glycosyltransferase of the present invention, by the enzyme encoded by the introduced gene. Thus, the host microorganism into which the nucleic acid encoding the C-glycosyltransferase variant and the type III polyketide synthase gene are introduced may have the ability to produce coenzyme A, preferably malonyl-CoA or acetyl-CoA.

Therefore, in the present invention, the recombinant microorganism may be characterized in that production of coenzyme A is enhanced. For example, in the present invention, the recombinant microorganism may be additionally characterized in that (ii) expression of the pabA gene is suppressed or attenuated, but the present invention is not limited thereto, and recombinant microorganisms with enhanced production of coenzyme A may be prepared using various strategies for mass production of coenzyme A known in the art.

In the present invention, the recombinant microorganism may be characterized by enhanced production of nucleotides, preferably NTP-sugar, in order to increase glycoside conversion efficiency by the introduced C-glycosyltransferase. For example, in the present invention, the recombinant microorganism may be additionally characterized in that (iii) expression of a gene encoding UTP-glucose-1-phosphate uridyltransferase, phosphoglucomutase, and/or nucleoside-diphosphate kinase is enhanced, but the present invention is not limited thereto.

In the present invention, the UTP-glucose-1-phosphate uridyltransferase, phosphoglucomutase, and/or nucleoside-diphosphate kinase may be derived from *E. coli,* but the present invention is not limited thereto, and expression of a gene involved in the production of NTP-sugar may be enhanced depending on the host strain.

In the present invention, the recombinant microorganism may be used for production of a phenylpropanoid glycoside. For example, the phenylpropanoid glycoside may be, but is not limited to, vitexin, naringenin-6-C-glucoside, or isoorientin.

In the present invention, the recombinant microorganism for producing the phenylpropanoid glycoside is able to produce a precursor of the phenylpropanoid glycoside. For example, the precursor of the phenylpropanoid glycoside may be, but is not limited to, apigenin, naringenin, or luteolin.

In the present invention, the recombinant microorganism for producing the phenylpropanoid glycoside is able to produce a precursor of the phenylpropanoid glycoside through additional gene introduction.

In the present invention, the recombinant microorganism for producing the phenylpropanoid glycoside may be characterized in that:
(i) a gene encoding phenylpropanoid synthase is additionally introduced thereto.

For the phenylpropanoid, coenzyme A (CoA) such as malonyl-CoA or aromatic-CoA (e.g. coumaroyl-CoA) may be converted into a phenylpropanoid, which is a substrate of the C-glycosyltransferase of the present invention, by the enzyme encoded by the introduced gene. Thus, the host microorganism into which the nucleic acid encoding the C-glycosyltransferase variant and the phenylpropanoid synthase gene are introduced may have the ability to produce coenzyme A, preferably malonyl-CoA or coumaroyl-CoA.

Therefore, in the present invention, the recombinant microorganism may be characterized in that production of coenzyme A is enhanced. For example, in the present invention, the recombinant microorganism may be additionally characterized in that (ii) expression of the pabA gene is suppressed or attenuated, but the present invention is not limited thereto, and recombinant microorganisms with enhanced production of coenzyme A may be prepared using various strategies for mass production of coenzyme A known in the art.

In the present invention, the recombinant microorganism may be characterized by enhanced production of nucleotides, preferably NTP-sugar, in order to increase glycoside conversion efficiency by the introduced C-glycosyltransferase. For example, in the present invention, the recombinant microorganism may be additionally characterized in that (iii) expression of a gene encoding UTP-glucose-1-phosphate uridylyltransferase, phosphoglucomutase, and/or nucleoside-diphosphate kinase is enhanced, but the present invention is not limited thereto.

In the present invention, the UTP-glucose-1-phosphate uridyltransferase, phosphoglucomutase, and/or nucleoside-diphosphate kinase may be derived from *E. coli,* but the present invention is limited thereto, and expression of a gene involved in the production of NTP-sugar may be enhanced depending on the host strain.

In an exemplary embodiment, the recombinant microorganism of the present invention may be a recombinant microorganism for producing carminic acid, in which at least one gene introduction or gene expression selected from the group consisting of the following is controlled in the recombinant microorganism into which the nucleic acid encoding the C-glycosyltransferase of the present invention is introduced:
(i) introduction of a gene encoding type II polyketide synthase;
(ii) introduction of a gene encoding 4'-phosphopantetheinyl transferase;
(iii) introduction of a gene encoding cyclase;
(iv) introduction of a gene encoding acetyl-CoA carboxylase;
(v) introduction of a gene encoding aklavinone 12-hydroxylase;
(vi) enhanced expression of a gene encoding UTP-glucose-1-phosphate uridylyltransferase, phosphoglucomutase, and/or nucleoside-diphosphate kinase; and
(vii) attenuated expression of a pabA gene.

In another exemplary embodiment, the recombinant microorganism of the present invention may be a recombinant microorganism for producing aloesin, in which at least one gene introduction or gene expression selected from the group consisting of the following is controlled in the recombinant microorganism into which the nucleic acid encoding the C-glycosyltransferase of the present invention is introduced:
(i) introduction of a gene encoding aloesone synthase;
(ii) attenuated expression of a pabA gene; and
(iii) enhanced expression of a gene encoding glucose 6-phosphate 1-dehydrogenase.

In still another exemplary embodiment, the recombinant microorganism of the present invention may be:
a recombinant microorganism for producing a polyketide glycoside or phenylpropanoid glycoside, in which expression of a gene encoding UTP-glucose-1-phosphate uridyltransferase, phosphoglucomutase, and/or nucleoside-diphosphate kinase is enhanced in the recombinant microorganism into which the nucleic acid encoding the C-glycosyltransferase of the present invention is introduced.

In the present invention, introduction of a gene means that a foreign gene is introduced into a host microorganism through a vehicle such as a vector, or is directly inserted into the genome of a host microorganism.

In the present invention, enhanced expression of a gene means that, when a peptide or protein produced by the gene is not present in the host microorganism, the activity or function of the peptide or protein is imparted through artificial expression in the host microorganism and also that, when the gene is already present in the host microorganism, an endogenous promoter that controls gene expression is replaced with a strong constitutive expression promoter or the gene is additionally introduced from the outside using a vector having strong replication ability, etc. to increase the copy number of the gene, thereby inducing overexpression of the gene or modifying the activity or function of the peptide or protein produced by the gene so as to be enhanced compared to intrinsic activity or function.

In the present invention, attenuated expression of a gene means that a relevant gene is not expressed by mutating, substituting, or deleting some or all bases of the relevant gene, or by introducing an inhibitor (e.g. sRNA, etc.) capable of suppressing gene expression, or does not show activity or function even when expressed, and is a concept encompassing modification so that the activity or function of the peptide or protein produced by the gene is attenuated compared to intrinsic activity or function.

As used herein, the term "intrinsic activity or function" refers to the activity or function of enzymes, peptides, proteins, etc. originally possessed by microorganisms in an unmodified state.

In the present invention, "modified so as to be enhanced compared to intrinsic activity or function" means that the activity or function of microorganisms after manipulation is newly generated or increased compared to the activity of microorganisms before manipulation, such as introduction of a gene exhibiting activity or function or an increase in the copy number of the gene (e.g. expression using a plasmid into which the gene is introduced), deletion of a suppressor of gene expression, or modification of an expression control sequence, for example use of an improved promoter, etc.

In the present invention, "modified so as to be attenuated compared to intrinsic activity or function" means that the function of microorganisms after manipulation is reduced or lost compared to the function of microorganisms before manipulation, such as deletion of a gene exhibiting activity or function or inactivation of a gene (e.g. substitution with a mutation gene), attenuation of gene expression (e.g. substitution with a weak promoter, introduction of siRNA, gRNA, sRNA, etc., substitution of the start codon from ATG to GTG, etc.), inhibition of the function of a peptide expressed by the gene (e.g. addition of a non-competitive repressor or competitive repressor), and the like.

In the present invention, "replacement" of a gene or promoter means removing a conventional gene or promoter and newly introducing a different gene (e.g. a mutation gene, etc.) or a promoter having different intensity, and removing the conventional gene or promoter is a concept encompassing not only deleting the corresponding gene or promoter, but also suppressing or reducing the function thereof.

As used herein, the term "overexpression" refers to a higher level of expression than the level at which a relevant gene is expressed in cells under normal conditions, and is a concept that includes increasing the expression level either by replacing the promoter of a gene present in the genome with a strong promoter or by cloning the relevant gene into an expression vector and transforming cells therewith.

As used herein, the term "vector" refers to a DNA construct containing a DNA sequence operably linked to suitable regulatory sequences capable of expressing DNA in a suitable host. The vector may be a plasmid, a phage particle, or simply a potential genomic insert. Once transformed into an appropriate host, the vector may replicate and function independently of the host genome, or in some cases may be integrated into the genome itself. As the plasmid is currently the most commonly used form of vector, "plasmid" and "vector" are sometimes used interchangeably in the context of the present invention. For the purposes of the present invention, it is preferred to use plasmid vectors. Typical plasmid vectors that may be used for this purpose may be configured to include (a) an origin of replication that allows efficient replication so as to include ones to hundreds of plasmid vectors per host cell, (b) an antibiotic resistance gene that allows selection of host cells transformed with the plasmid vector, and (c) a restriction enzyme cleavage site into which a foreign DNA fragment may be inserted. Even in the absence of an appropriate restriction enzyme cleavage site, the vector and foreign DNA may be easily ligated using a synthetic oligonucleotide adapter or linker according to a typical method. After ligation, the vector has to be transformed into an appropriate host cell. Transformation may be easily achieved using a calcium chloride method or electroporation (Neumann, et al., EMBO J., 1:841, 1982).

The promoter of the vector may be constitutive or inducible, and may be additionally modified for the effect of the present invention. Also, the expression vector includes a selection marker for selecting a host cell containing the vector, and in the case of a replicable expression vector, an origin of replication (Ori) is included. Vectors may replicate autonomously or may be integrated into the host genomic DNA. It is preferred that the gene inserted and delivered into the vector be irreversibly fused into the genome of the host cell so that gene expression is stably maintained for a long period of time in the cell.

A base sequence is "operably linked" when placed in a functional relationship with another nucleic acid sequence. This may be a gene and regulatory sequence(s) linked in such a way as to enable gene expression when an appropriate molecule (e.g. a transcriptional activating protein) binds to the regulatory sequence(s). For example, DNA for a pre-sequence or secretory leader is operably linked to DNA for a polypeptide when expressed as a pre-protein that participates in secretion of the polypeptide; a promoter or enhancer is operably linked to a coding sequence when affecting transcription of the sequence; a ribosome binding site is operably linked to a coding sequence when affecting transcription of the sequence; or a ribosome binding site is operably linked to a coding sequence when arranged to facilitate translation. Generally, "operably linked" means that the DNA sequences being linked are contiguous and, in the case of a secretory leader, are contiguous and present in the reading frame. However, enhancers need not be contiguous. Linking of these sequences is accomplished by ligation at convenient restriction enzyme sites. When such a site does not exist, a synthetic oligonucleotide adapter or linker according to a typical method is used.

As is well known in the art, in order to increase the expression level of a transgene in a host cell, the gene has to be operably linked to transcriptional and/or translational expression control sequences that function in the selected expression host. Preferably, the expression control sequence and/or the corresponding gene are contained in a single recombinant vector that includes both a bacterial selection marker and a replication origin. When the host cell is a eukaryotic cell, the recombinant vector must further include an expression marker useful in the eukaryotic expression host.

A further aspect of the present invention pertains to a host cell transformed with the recombinant vector described above. As used herein, the term "transformation" means introducing DNA into a host so that the DNA becomes replicable as an extrachromosomal factor or by completion of chromosomal integration.

It is to be understood that not all vectors function equally well in expressing the DNA sequences of the present invention. Likewise, not all hosts function equally for the same expression system. However, those skilled in the art will be able to make an appropriate selection among various vectors, expression control sequences, and hosts without departing from the scope of the present invention without undue experimentation. For example, in selecting a vector, the host must be considered because the vector must be replicated therein.

Still a further aspect of the present invention pertains to a method of producing a polyketide glycoside or a phenylpropanoid glycoside, including:
(a) producing a polyketide glycoside or phenylpropanoid glycoside by culturing a recombinant microorganism into which the nucleic acid encoding the C-glycosyltransferase variant of the present invention is introduced; and
(b) recovering the produced polyketide glycoside or phenylpropanoid glycoside.

In the present invention, step (a) may be performed in a manner in which the recombinant microorganism into which the nucleic acid encoding the C-glycosyltransferase variant is introduced is cultured by adding a precursor of the polyketide glycoside or phenylpropanoid glycoside.

In the present invention, in step (a), the recombinant microorganism into which the nucleic acid encoding the C-glycosyltransferase variant is introduced may be configured such that the nucleic acid encoding the C-glycosyltransferase variant is introduced into a host microorganism capable of producing a precursor of the polyketide glycoside or phenylpropanoid glycoside, and the host microorganism may be a recombinant microorganism into which a foreign gene is introduced or in which gene expression is controlled.

In the present invention, the recombinant microorganism into which the nucleic acid encoding the C-glycosyltransferase variant is introduced may have the same characteristics as those described in the recombinant microorganism for producing the polyketide glycoside and/or phenylpropanoid glycoside according to the present invention.

In the present invention, step (a) may be performed in a manner in which the microorganism is cultured by adding ascorbic acid to a culture medium during culture. Here, the microorganism may be cultured by adding preferably 0.1 to 1.5 g/L, more preferably 0.2 to 1.0 g/L of ascorbic acid, but the present invention is not limited thereto.

Unless otherwise described, the production method of the present invention may have the same characteristics as those described in the other aspects within a range that may be understood by a person skilled in the art.

Yet a further aspect of the present invention pertains to a method of producing a polyketide glycoside and/or a phenylpropanoid glycoside, including:
(a) producing a polyketide glycoside and/or phenylpropanoid glycoside by reacting the C-glycosyltransferase variant of the present invention or a microorganism expressing the C-glycosyltransferase variant with a polyketide and/or phenylpropanoid; and
(b) recovering the produced polyketide glycoside and/or phenylpropanoid glycoside.

Although specific gene names are described in the present invention, it will be apparent to those skilled in the art that the present invention is not limited to the corresponding genes.

Moreover, even in the gene introduced in the present invention, the gene name derived from a specific microorganism is described, but the scope of protection of the present invention is not limited to the corresponding gene name, and within a range that a person skilled in the art may recognize as having the same function as the corresponding gene, when a gene derived from another microorganism having a different gene name is introduced according to the technical features of the present invention, it is obvious that the corresponding recombinant microorganism may also fall within the scope of protection of the present invention.

### Examples

A better understanding of the present invention may be obtained through the following examples. However, these examples are not to be construed as limiting the present invention, and may be variously changed or altered within the idea and scope of the present invention, as is obvious to those skilled in the art.

### Example 1. Experimental method

### 1-1. Flask culture

Flask culture was carried out under the following conditions. Colonies were seeded into 10 mL of LB medium supplemented with appropriate concentrations of antibiotics, followed by culture overnight at 37°C. The culture fluid thus prepared was passaged into a 250 mL baffled flask containing 50 mL of R/2 medium supplemented with 3 g/L yeast extract and 20 g/L glucose (with 0.45 g/L ascorbic acid if necessary), followed by culture at 30°C and 200 rpm. The composition of R/2 medium (pH 6.8) was as follows (per liter): 2 g of (NH₄)₂HPO₄, 6.75 g of KH₂PO₄, 0.85 g of citric acid, 0.7 g of MgSO₄·7H₂O, and 5 ml of trace metal solution (TMS) [10 g of FeSO₄·7H₂O, 2.25 g of ZnSO₄·7H₂O, 1 g of CuSO₄·5H₂O, 0.5 g of MnSO₄·5H₂O, 0.23 g of Na₂B₄O₇·10H₂O, 2 g of CaCl₂·2H₂O, and 0.1 g of (NH₄)₆Mo₇O₂₄ per liter of 5 M HCl]. When OD600 of the culture fluid reached 0.6-0.8, 1 mM isopropyl β-D-1-thiogalactopyranoside (IPTG) was added to induce exogenous gene expression, followed by culture for 48 hours.

### 1-2. Fed-batch fermentation

Fed-batch fermentation was carried out in 1.95 L of R/2 medium (pH 6.8) containing 20 g/L glucose, 3 g/L yeast extract, 0.45 g/L ascorbic acid, and appropriate antibiotics using a 6.6 L BioFlo 320 fermenter (Eppendorf). Colonies were seeded into 10 mL of LB medium supplemented with appropriate concentrations of antibiotics, followed by culture overnight at 37°C. The culture fluid thus prepared was passaged into a 250 mL baffled flask containing 50 mL of R/2 medium supplemented with 3 g/L yeast extract, 20 g/L glucose, and 0.45 g/L ascorbic acid, followed by culture at 30°C and 200 rpm until OD600 reached about 4. After seeding using a fermenter, the pH was maintained at 6.8 through automatic addition of ammonia solution, and the temperature was maintained at 30°C. Oxygen saturation (DO) was maintained at 40% of the air saturation level, and 1 vvm [(air volume)·(working volume)⁻¹·(minute)⁻¹] of air was continuously blown, provided that DO was maintained by increasing the stirring speed or the concentration of pure oxygen that was added. IPTG (0.5 mM) was added when OD600 reached about 20, and the depleted carbon source and other nutrients were automatically added into the fermentor through pH-stat strategy. As such, the addition liquid contained the following components per liter: 650 g of glucose, 5 g of ascorbic acid, 6 mL of TMS, and 8 g of MgSO₄·7H₂O. When the pH was higher than 6.85, the addition liquid was automatically added to the fermenter.

### 1-3. Production analysis

After culture, production analysis was performed under the following conditions. After flask culture, 50 mL of the culture fluid was centrifuged at 4,000 g for 30 minutes, and then the supernatant was desalted and concentrated. Here, Oasis HLB cartridges (Water) were used. The supernatant was concentrated 1-fold for FK, 30-fold for KA, 45-fold for dcII, and 200-fold for carminic acid. The concentrated sample was re-dissolved in an appropriate volume of DMSO and impurities were then removed therefrom using a 0.22 um PTFE filter. The prepared sample was analyzed by MS (LC/MSD VL; Agilent) coupled with HPLC (1100 series; Agilent). An Eclipse XDB-C18 column was used, 0.1% formic acid was used as buffer A, and methanol was used as buffer B. Analysis was performed in an ESI negative mode. For more accurate analysis of carminic acid, LC-MS/MS was performed using an HPLC Triple Quadrupole Mass Spectrometer (LCMS-8050, Shimadzu) (MRM mode).

Also, in LC-MS/MS for aloesin analysis, MS (Agilent 6550 iFunnel Q-TOF LC/MS System) coupled with ultra HPLC (UHPLC; 1290 Infinity II LC System; Agilent) was used. Here, an Eclipse-plus C18 column was used, and 0.1% formic acid was used as buffer A, and acetonitrile to which 0.1% formic acid was added was used as buffer B.

### Example 2: Identification of C-glycosyltransferase for production of carminic acid

Although the production pathway of carminic acid has not yet been specifically identified, since the carbon skeleton of carminic acid has an anthraquinone-based structure, production of carminic acid was induced using PKS (FIG. 1).

Accordingly, the production method using an *E. coli* BAP1 strain *(E. coli* BL21(DE3) (Invitrogen)) into which Sfp from *Bacillus subtilis* was introduced into the genome for the activity of exogenous acyl carrier protein (ACP) was performed with reference to B. Pfeifer et al., Science 2001, 291 (5509), 1790-1792 / D. Yang et al., PNAS 2018, 115(40) 9835-9844. Thereafter, in order to apply type II PKS from *Photorhabdus luminescens,* pDS00-antDEFBG was constructed to introduce P. *luminescens-derived antD* (ketosynthase), *antE* (chain-length factor), *antF* (ACP), *antB* (phosphopantetheinyl transferase), and *antG* (malonyl-CoA:ACP malonyl transferase). Specifically, the *antDEF* gene was amplified by PCR using the antDE_F primer and the antDEF_R primer from genomic DNA of *P. luminescens,* and then inserted into NdeI and EcoRI restriction enzyme sites of pDS00 (a platform plasmid identical to the pET-30a(+) primer except for the restriction enzyme sequence, Novagen). The pDS00 plasmid was constructed as follows. The gene fragment containing the T7 promoter, multiple cloning site (MCS), and T7 terminator was amplified from pET-30a(+) using pET_NheI_DraIII and pET_SpeI_SphI primers, treated with SphI and DraIII restriction enzymes, and inserted into the SphI and DraIII sites of the pET-30a(+) plasmid to construct a pDS00 plasmid. Thereafter, *antB* was amplified from genomic DNA of P. *luminescens* using the antB_F primer and the antB_R primer and inserted into the HindIII site of pDS00 to construct a pDS00-antB plasmid. Subsequently, after digestion using NdeI and EcoRI restriction enzymes, the pDS00-antDEF plasmid was also digested using NdeI and EcoRI restriction enzymes to obtain two antDEF fragments, which were then combined using Gibson assembly, yielding a pDS00-antDEFB plasmid. Also, *antG* was amplified from genomic DNA of *P. luminescens* using the antG_F primer and the antG_R primer, and inserted into NdeI and EcoRI sites of pDS00 to construct a pDS00-antG plasmid. The constructed plasmid was digested with NheI and SpeI restriction enzymes to obtain an *antG* fragment, which was then inserted into the NheI site of the pDS00-antDEFB plasmid to construct a pDS00-antDEFBG plasmid.

**[Table 1]**

| Name | Sequence | SEQ ID NO: |
|---|---|---|
| pET_NheI_DraII I primer (forward) | 5'-AAACACTACGTGGCTAGCCAAAAAACCCCTCAAGACC-3' | 3 |
| pET_SpeI_SphI primer (reverse) | 5'-AAAGCATGCACTAGTTAATACGACTCACTATAGGG-3' | 4 |
| antDE_F primer(Forward ) | 5′- CTTTAAGAAGGAGATATACATATGATAATAAATAACAGAAATGAAT C-3′ | 5 |
| antDEF_R primer(Reverse ) | 5′- CTTGTCGACGGAGCTCGAATTCATTAATTTTTATCGTTTAAACTTG ATG -3′ | 6 |
| antB F primer (Forward) | 5′- GAATTCGAGCTCCGTCGACAAATAAGGAGATATACCATGGACGATA TTTCTTTATCATCTG -3′ | 7 |
| antB_R primer (Reverse) | 5′- GTGGTGCTCGAGTGCGGCCGCAAGCTTATTACTCATCTTTGTTCCT TATAATC -3′ | 8 |
| antG_F primer (Forward) | 5′- CTTTAAGAAGGAGATATACATATGAAACTAATCTCTATGTTGTTAC -3′ | 9 |
| antG_R primer (Reverse) | 5′- CTTGTCGACGGAGCTCGAATTCATTATTGATTCCTCAATGTAAATA G -3′ | 10 |

In order to produce flavokermesic acid (FK), ZhuI and ZhuJ, which are cyclases from *Streptomyces sp.* R1128, were introduced. To this end, a pFK (pDS00-antDEFBG-zhuIJ) plasmid was constructed. Specifically, a *zhuIJ* fragment was amplified by PCR using the zhuI_F primer and the zhuJ_R primer based on codon-optimized *zhuIJ* DNA for expression in E. *coli,* and inserted into NdeI and EcoRI sites of pDS00. The pDS00-zhuIJ thus constructed was digested with NheI and SpeI restriction enzymes to obtain a *zhuIJ* fragment, which was then inserted into the NheI site of pDS00-antDEFBG to construct pFK.

The strain in which BAP1 was transformed with pDS00-antDEFBG-zhuIJ produced 88 mg/L of FK from glucose. It was observed that the color of the culture fluid was bright red at the beginning of culture and changed to turbid brown with time. This was deemed to be due to conversion of FK into melanin analogues, etc., and thus, 0.45 g/L of ascorbic acid was added to the medium in order to prevent melanization of FK, thereby increasing FK production to 154.9 mg/L.

**[Table 2]**

| Name | Sequence | SEQ ID NO: |
|---|---|---|
| zhuI_F primer (Forward) | 5′- CTTTAAGAAGGAGATATACATATGCGTCATGTAGAGCATACAGTC - 3′ | 11 |
| zhuJ_R primer (Reverse) | 5′- CTTGTCGACGGAGCTCGAATTCTTATTAATCCTCTTCCTCTTGCTCG -3′ | 12 |

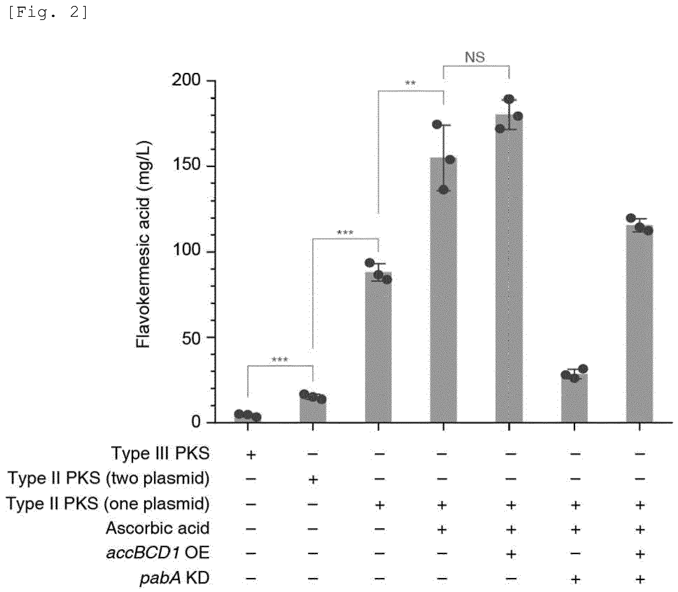

Increasing the intracellular concentration of malonyl-CoA was also predicted as a way to increase FK production, and acetyl-CoA carboxylase from *Corynebacterium glutamicum* (encoded by the *accBCD1* gene) was overexpressed or the *pabA* gene was knocked down. Thereby, in the strain overexpressing *accBCD1,* FK production was increased to 180.3 mg/L (FIG. 2).

Flask culture started by seeding colonies from LB agar plates into test tubes containing 10 mL of LB. As such, an appropriate concentration of antibiotics was added, followed by culture overnight at 37°C at 220 rpm. 1 mL of the seed culture thus prepared was inoculated into a 250 mL baffled flask containing 50 mL of R/2 medium (supplemented with 3 g/L yeast extract and 20 g/L glucose), followed by culture at 30°C and 200 rpm. The composition of R/2 medium was as follows (pH 6.8, per 1 L) : 2 g of (NH₄)₂HPO₄, 6.75 g of KH₂PO₄, 0.85 g of citric acid, 0.8 g of MgSO₄·7H₂O, and 5 ml of trace metal solution (TMS). The composition of TMS was as follows (based on 0.1 M HCl, per 1 L): 10 g of FeSO₄·7H₂O, 2.25 g of ZnSO₄·7H₂O, 1 g of CuSO₄·5H₂O, 0.58 g of MnSO₄·5H₂O, 0.02 g of Na₂B₄O₇·10H₂O, 2 g of CaCl₂·2H₂O, and 0.1 g of (NH₄)₆Mo₇O₂₄·4H₂O. When OD600 of the culture fluid reached 0.6-0.8, 0.5 mM IPTG was added to induce exogenous enzyme expression, followed by culture for 48 hours. In all experiments for producing flavokermesic acid, kermesic acid, dcII, and carminic acid, 0.45 g/L of ascorbic acid was further added.

Based on results of flask culture, a small amount of kermesic acid (KA) was also observed (0.14 mg FK equivalent/L; i.e. mg FK eq/L). This was thought to be caused by *E. coli* endogenous oxidase or ZhuIJ, but the conversion efficiency was too low and the enzyme responsible for the relevant reaction had not yet been identified. In the present invention, biochemical reaction analysis was performed using previously reported literature and compound databases.

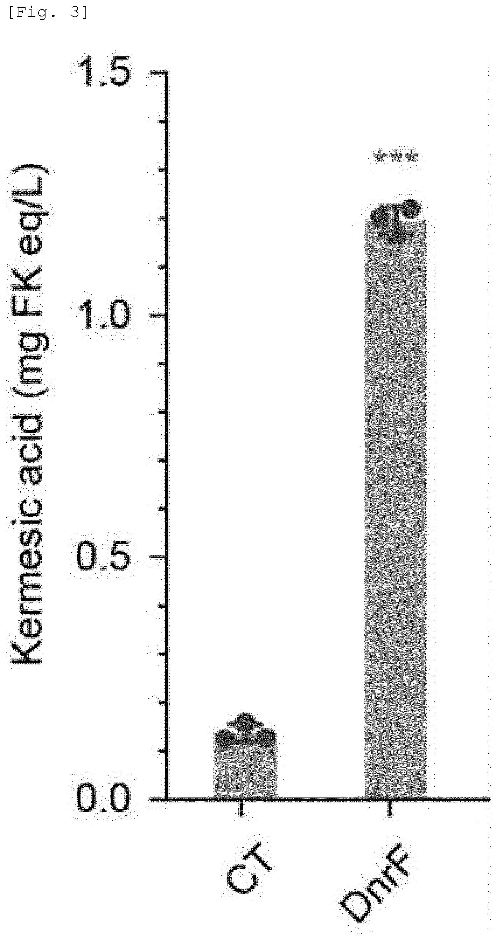

Consequently, the gene encoding aklavinone 12-hydroxylase (DnrF) from *Streptomyces peucetius* was predicted to be an enzyme that performs the relevant reaction, and was amplified using the dnrF_F primer and the dnrF_R primer and then inserted into NdeI and EcoRI sites of pDS00 to construct pDS00-dnrF. The *dnrF* gene was amplified by PCR using the pET30a_frag_F primer and the pET30a_frag_R primer based on the corresponding plasmid, and the pBBR1-T7 plasmid (constructed by the method of S.Y.Park et al., bioRxiv, DOI: 10.1101/2020.11.27.401000 from Kovach, M. E.; Phillips, R. W.; Elzer, P. H.; Roop, R. M., II; Peterson, K. M., pBBR1MCS: a broad-host-range cloning vector. Biotechniques 1994, 16 (5), 800-802) was amplified by reverse PCR using the pET30a_IV_R primer and the rrnB_IV_F primer, and two DNA fragments were ligated using Gibson assembly to construct pBBR1-dnrF. After introducing pBBR1-dnrF into the FK-producing strain, flask culture was carried out. Thereby, 1.20 mg FK eq/L of KA was produced (FIG. 3a).

**[Table 3]**

| Name | Sequence | SEQ ID NO: |
|---|---|---|
| dnrF_F primer (Forward) | 5′- CTTTAAGAAGGAGATATACATATGGCCTTGACGAAGCCGGATG - 3′ | 13 |
| dnrF_R primer (Reverse) | 5′- CAAGCTTGTCGACGGAGCTCG AGTTGTCGGAGCGGCTGGCC -3′ | 14 |
| pET30a frag_F primer (Forward) | 5'- CTTTAAGAAGGAGATATACATATG -3' | 15 |
| pET30a frag_R primer (Reverse) | 5′- CAAAACAGCCAAGCTTGCATG CAAGCTTGTCGACGGAGCTC -3′ | 16 |
| rrnB_IV_F primer (Forward) | 5'- CATGCAAGCTTGGCTGTTTTG -3' | 17 |
| pET30a_IV_R primer | 5'- CATATGTATATCTCCTTCTTAAAG -3' | 18 |
| (Reverse) | | |

The production of carminic acid (CA) from FK was performed by two biosynthesis pathways, both of which require monooxygenase and C-glycosyltransferase. FK may be oxidized into KA or may be C-glycosylated into dcII. DcUGT2 from D. *coccus* has been revealed to catalyze the conversion from FK to dcII (or from KA to CA), and activity thereof has been demonstrated in *S. cerevisiae* (Kannangara et al., Nat Commun 2017, 8). In order for the enzyme to have activity, however, it must be glycosylated, and since it also has a transmembrane helix and a signal peptide, successful expression in cells such as *E. coli* is considered difficult. Indeed, DcUGT2 failed to produce dcII when introduced into FK-producing *E. coli.* In order to solve the problem related to DcUGT2 expression, Ntr-DcUGT2 with the N-terminal signal peptide removed, Ctr-DcUGT2 with the C-terminal transmembrane helix removed, and Ntr-Ctr-DcUGT2 with both the N-terminal signal peptide and the C-terminal transmembrane helix removed were constructed, and plasmids with *E. coli* OmpA signal peptide attached to the N-terminus of Ntr-DcUGT2 and Ntr-Ctr-DcUGT2 were also constructed, but all failed to produce dcII. Therefore, it was concluded that DcUGT2 has no activity in *E. coli.*

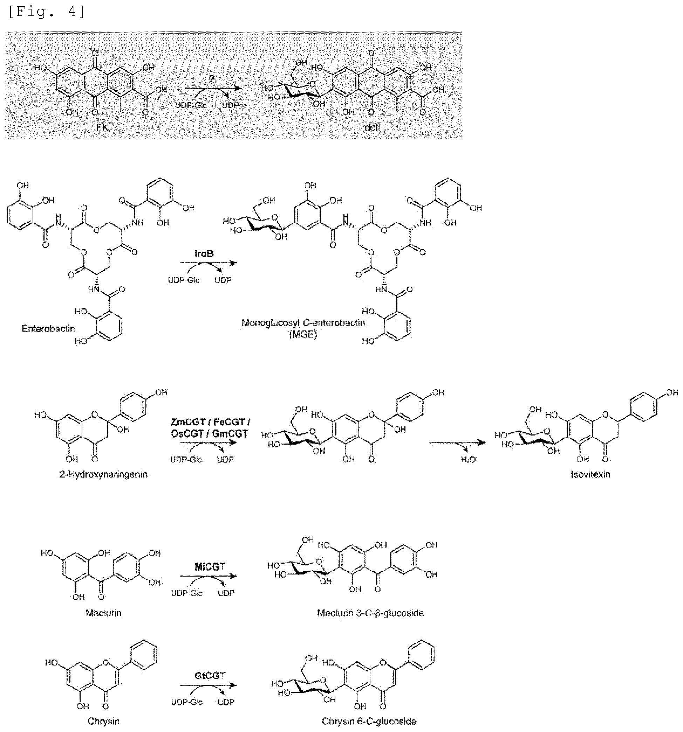

A few cases of O-glycosylation of natural products have been reported in *E*. *coli,* but almost no cases of C-glycosylation have been reported. Hence, in the present invention, UDP-glycosyltransferase, which was found to carry out C-glycosylation reaction in *E. coli* through biochemical reaction analysis, was selected. The eight enzyme candidates selected were as follows: IroB (EnCGT) from *E. coli* Nissle; UGT708A6 (ZmCGT) from *Zea mays* which acts as a dual C/O-glycosyltransferase; UGT708C2 (FeCGT) from *Fagopyrum esculentum;* MiCGT from *Mangifera indica;* OsCGT from *Oryza sativa;* UGT708D1 (GmCGT) from *Glycine max;* GtUF6CGT1 (GtCGT) from *Gentiana triflora;* and AvCGT from *Aloe vera* (FIG. 4).

For the enzymes selected above, pCDF-DcCGT, pCDF-MiCGT, pCDF-SfCGT, pCDF-EnCGT, pCDF-OsCGT, pCDF-FeCGT, pCDF-GmCGT, pCDF-AvCGT, pCDF-AvCGT, pCDF-ZmCGT, and pCDF-GtCGT were constructed, and all genes except for the *iroB* gene amplified from genomic DNA of *E. coli* Nissle using the iroB_gib_F primer and the iroB_gib_R primer were artificially synthesized and inserted into the NdeI site of the pCDFDuet-1 plasmid using Gibson assembly.

**[Table 4]**

| Name | Sequence | SEQ ID NO: |
|---|---|---|
| iroB_gib_F primer (Forward) | 5′- GTTAAGTATAAGAAGGAGATATACATATGCGTCGACTGCCTG ATTTAG -3′ | 19 |
| iroB_gib_R primer (Reverse) | 5′- GATATCCAATTGAGATCTGCCTTATGATTACCCTTTCTGTAC CATTGTGATC -3′ | 20 |

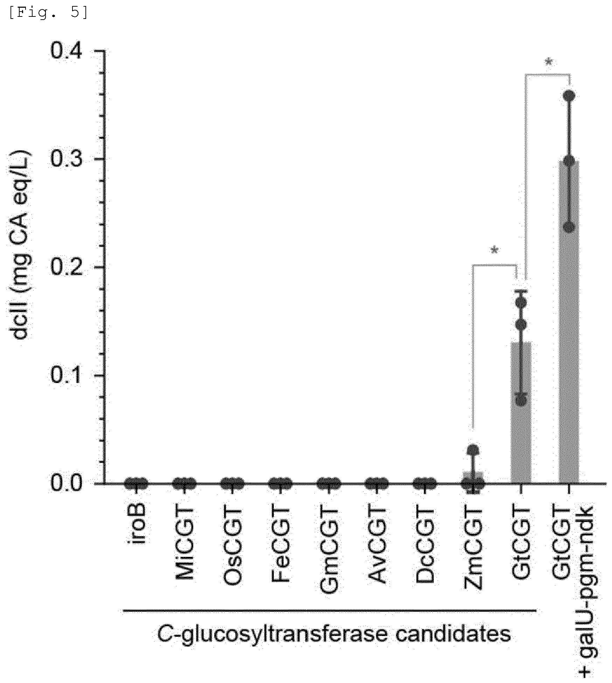

Only GtCGT and ZmCGT were able to successfully convert FK into dcII. For ZmCGT, the main product was O-glycosylated FK, and dcII was produced in a very small amount. For GtCGT, 0.13 mg CA equivalent/L (mg CA eq/L) of dcII was produced (FIG. 5). Since C-glycosylation requires a large amount of UDP-glucose, *galU* (encoding UTP-glucose-1-phosphate uridylyltransferase), *pgm* (encoding phosphoglucomutase), and *ndk* (encoding nucleoside-diphosphate kinase) were overexpressed, whereby dcII production was increased to 0.30 mg CA eq/L (FIG. 5). In order to construct the pBBR1-galU-pgm-ndk plasmid (all three genes were amplified from an *E. coli* BL21 (DE3) strain), the *galU* gene was amplified from galU_gib_F and galU_gib_R primers and inserted into the EcoRI site of the pBBR1TaC plasmid through Gibson assembly. The *pgm* gene was amplified from pgm_gib_F and pgm_gib_R primers and inserted into the KpnI site of the pBBR1TaC-galU plasmid, and the *ndk* gene was amplified from ndk_gib_F and ndk_gib_R primers and inserted into the SphI site of the pBBR1-galU-pgm plasmid, thereby constructing pBBR1-galU-pgm-ndk.

**[Table 5]**

| Name | Sequence | SEQ ID NO: |
|---|---|---|
| galU_Gib_F primer (Forward) | 5′- CAATTTCACACAGGAAACAGAATTCATGGCTGCCATTAATACGAAAG -3′ | 21 |
| galU_Gib_R primer (Reverse) | 5'- CCGGGTACCGAGCTCGAATTC ATTACTTCTTAATGCCCATCTC -3' | 22 |
| pgm_Gib_F primer (Forward) | 5′- GTAATGAATTCGAGCTCGGTACCCAATTTCACACAGGAAACAGA ATGGCAATCCACAATCGTGC -3′ | 23 |
| pgm_Gib_R primer (Reverse) | 5′- CTCTAGAGGATCCCCGGGTACCATTACGCGTTTTTCAGAACTTC - 3′ | 24 |
| ndk_Gib_F primer (Forward) | 5′- CTAGAGTCGACCTGCAGGCATGC CAATTTCACACAGGAAACAGA ATGGCTATTGAACGTACTTTTTCC -3′ | 25 |
| ndk_Gib_R primer (Reverse) | 5′- CAAAACAGCCAAGCTTGCATGC ATTAACGGGTGCGCGGGCAC -3′ | 26 |

In order to successfully produce carminic acid by increasing the activities of GtCGT and DnrF, the present inventors attempted to create mutants with increased activity through computer simulation. However, since the structures of the relevant enzymes have not been revealed, protein structures were analyzed using MODELLER (Webb, B.; Sali, A., Comparative protein structure modeling using MODELLER. Curr. Protoc. Bioinformatics 2016, 54, 5.6.1-5.6.37). Thereafter, mutants with increased activity were screened using docking simulation through PyRosetta (Chaudhury, S.; Lyskov, S.; Gray, J. J., PyRosetta: a script-based interface for implementing molecular modeling algorithms using Rosetta. Bioinformatics 2010, 26 (5), 689-691). Mutants expected to increase activity were additionally selected based on structural analysis results in addition to computer simulation-based predictions.

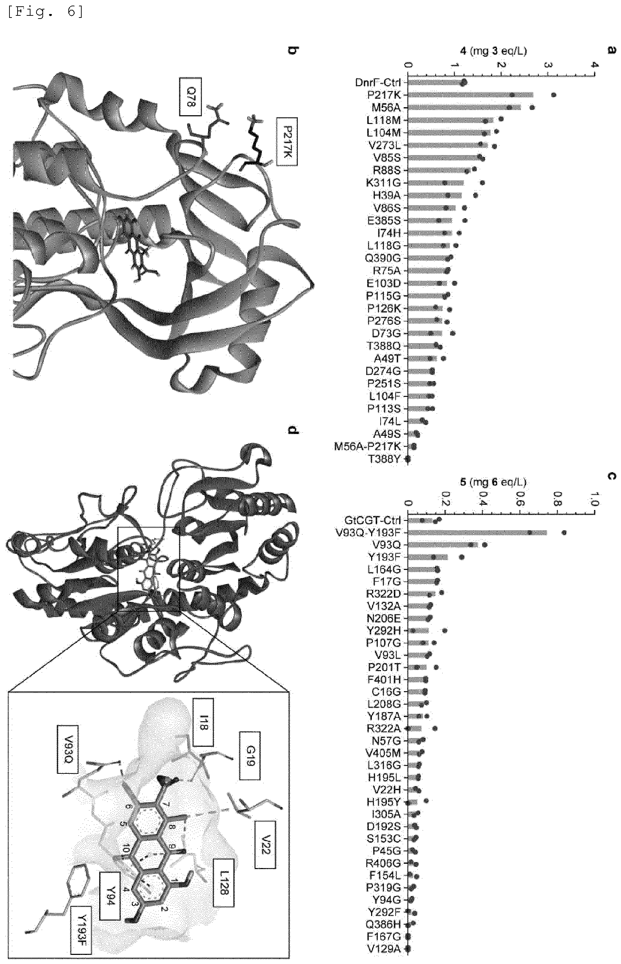

Homology modeling for GtCGT was performed using TcCGT (C-glycosyltransferase from *Trollius chinensis;* PDB ID 6JTD; protein sequence similarity of 35.1%) as a comparison group. Computer-based docking simulation (SW: AutoDock Vina) using FK as a ligand was performed using the resultant GtCGT structural model. Thereby, 239 mutants were generated, among which 122 mutants showed higher docking scores than the wild-type enzyme (Table 6). Experimentation was performed on the top 20 mutants thereamong, and 14 mutants expected to increase the activity based on results of the predicted structural analysis of GtCGT were additionally tested. Based on results of flask culture after transformation of 34 mutants into FK strains, six mutants with higher KA production than wild-type GtCGT were selected (V93Q, Y193F, L164G, F17G, R322D, and V132A). Thereamong, the mutant that showed the highest dcII production was determined to be GtCGT^{V93Q}, and the production was increased about 2.9-fold (FIG. 6c). In this mutant, it was judged that the Gln93 amino acid was located at the active site and thus directly bound to FK. The Gln93 amino acid forms a hydrogen bond with the hydroxyl group of C6, which is predicted to orient the FK ligand correctly for C-glycosylation at C2. The Y193F mutant showed the second highest concentration of dcII. In order to evaluate the synergistic effect between these two mutants, a double mutant was constructed (GtCGT^{V93Q/Y193F}) and then introduced into the FK strain. The double mutant produced 0.74 mg CA eq/L of dcII, which is a 5.3-fold increase compared to wild-type GtCGT (FIG. 6c). In the V93Q mutant, the Tyr193 amino acid forms a hydrogen bond with the carbonyl group of C10 and prevents Gln93 from forming a hydrogen bond with the hydroxyl group of C6. Therefore, it is assumed that FK ligand binding is improved by replacing Tyr193 with Phe193 and inhibiting hydrogen bonding at C10 (FIG. 6d).

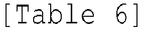

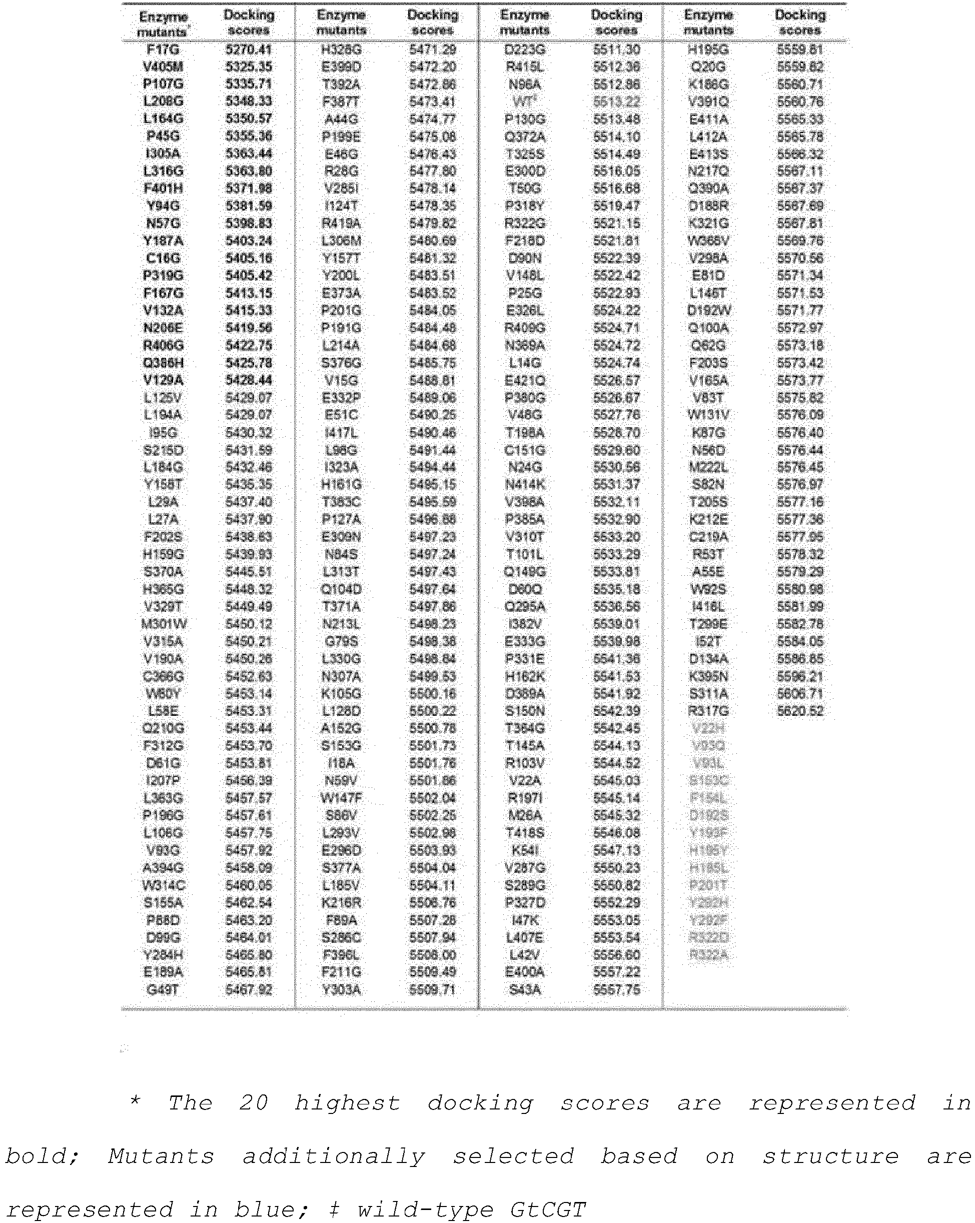

A mutant library was constructed using the same method for DnrF, based on which the mutant showing the highest KA production was determined to be DnrF^{P217K}, and the KA production was increased about 2.2-fold (2.68 mg FK eq/L) (FIGs. 6a and 6b) .

Mutagenesis for a specific sequence proceeded as reported in existing literature (Zheng, L.; Baumann, U.; Reymond, J. L., An efficient one-step site-directed and site-saturation mutagenesis protocol. Nucleic Acids Res 2004, 32(14), e115). Here, the plasmid pBBR1-T7 into which *dnrF*^{P217K} was introduced was named pKA, and the plasmid pCDFDuet-1 into which *GtCGT*^{V93Q/Y193F} was introduced was named pdcII. The P217K mutant of DnrF was constructed using the DnrF_P217K_F primer and the DnrF_P217K_R primer, the V93Q mutant of GtCGT was constructed using the GtCGT_V93Q_F primer and the GtCGT_V93Q_R primer, and the Y193F mutant of GtCGT was constructed using the GtCGT_Y193F_F primer and the GtCGT_Y193F_R primer.

**[Table 7]**

| Name | Sequence | SEQ ID NO: |
|---|---|---|
| DnrF_P217K_F primer (Forward) | 5'- gtcgtaccgAAGgggtccaccggctggtac-3' | 27 |
| DnrF_P217K_R primer (Reverse) | 5'- ggtggacccCTTcggtacgacggcggtgag -3' | 28 |
| GtCGT_V93Q_F primer (Forward) | 5'- gacgtatggCAAtacatcaatcacttagac -3' | 29 |
| GtCGT_V93Q_R primer (Reverse) | 5'- gattgatgtaTTGccatacgtcaaacggtt-3' | 30 |
| GtCGT_Y193F_F primer (Forward) | 5'- gtgcccgacTTCctgcatccgcgcacaccc-3' | 31 |
| GtCGT_Y193F_R primer (Reverse) | 5'- cggatgcagGAAgtcgggcacttcatcata -3' | 32 |

The protein sequence of the GtCGTV93Q/Y193F (GtUF6CGT1V93Q/Y193F) mutant constructed in the present invention is described below.

MGSLTNNDNLHIFLVCFIGQGVVNPMLRLGKAFASKGLLVTLSAPEIVGTEIRKA
NNLNDDQPIKVGSGMIRFEFFDDGWESVNGSKPFDVWQYINHLDQTGRQKLPIMLKKHEETGT
PVSCLILNPLVPWVADVADSLQIPCATLWVQSCASFSAYYHYHHGLVPFPTESEPEIDVQLPG
MPLLKYDEVPDFLHPRTPYPFFGTNILGQFKNLSKNFCILMDTFYELEHEIIDNMCKLCPIKP
IGPLFKIPKDPSSNGITGNFMKVDDCKEWLDSRPTSTVVYVSVGSVVYLKQEQVTEMAYGILN
SEVSFLWVLRPPSKRIGTEPHVLPEEFWEKAGDRGKVVQWSPQEQVLAHPATVGFLTHCGWNS
TQEAISSGVPVITFPQFGDQVTNAKFLVEEFKVGVRLGRGELENRIITRDEVERALREITSGP
KAEEVKENALKWKKKAEETVAKGGYSERNLVGFIEEVARKTGTK

After construction of the DnrF and GtCGT mutants with increased activity as described above, a CA strain was constructed by combining these two mutant enzymes. The CA strain was constructed by transforming the pFK and pCA (pCDF-dnrF^{P217K}-GtCGT^{V93Q/Y193F}) plasmids into the BAP1 strain. As such, in order to insert two genes into one plasmid, *dnrF*^{P217K} was amplified from pKA through PCR amplification using dnrF_NcoI_F and dnrF_BamHI_R primers, and then inserted into NcoI and BamHI sites of pdcII to construct pCA. Based on results of flask culture of the CA strain thus constructed, 22.2 µg/L of carminic acid was produced (FIG. 7). The carminic acid produced from glucose was identified through LC-MS/MS as shown in FIG. 7.

In order to increase the carminic acid production capacity, *C. glutamicum accBCD1* overexpression, *pabA* knockdown, and *galU-pgm-ndk* overexpression were tested individually or in combination, and the production in each strain was as follows (FIG. 7a): pabA KD, 25.9 µg/L; accBCD1 OE, 74.9 µg/L; galU-pgm-ndk OE, 41.0 µg/L; accBCD1 OE-galU-pgm-ndk OE, 49.9 µg/L; pabA KD-galU-pgm-ndk OE, 57.7 µg/L; pabA KD-accBCD1 OE, 57.2 µg/L; and pabA KD-accBCD1 OE-galU-pgm-ndk OE, 25.2 µg/L, confirming that carminic acid was produced in the highest concentration of 74.9 µg/L in the strain overexpressing *accBCD1* (BL21(DE3) harboring pFK, pCA, pACC; pFK: pDS00 derivative containing *antDEFBG* from *P. luminescens* and codon optimized *zhuIJ* from *Streptomyces sp.* R1128 (P_{T7} - *antDEFBG* - *T7*_{T} - P_{T7} - *zhuIJ -*

*T7*_{T}); pCA: pCDFDuet-1 derivative containing *dnrF*^{P217K} and *GtCGT*^{V93Q/Y193F} in different operons (P_{T7} - *dnrF*^{P217K} - *T7*_{T} - P_{T7} - *GtCGT*^{V93Q/Y193F} - *T7*_{T}); pACC: pBBR1TaC derivative containing *accBC* and *accDl* from *Corynebacterium glutamicum* ATCC 13032). Moreover, fed-batch fermentation of the strain was performed and thereby 0.65 mg/L of carminic acid was produced.

**[Table 8]**

| Name | Sequence | SEQ ID NO: |
|---|---|---|
| dnrF_NcoI_F primer (Forward) | 5′- CTTTAATAAGGAGATATACCATGGCCTTGACGAAGCCGGA TG -3′ | 33 |
| dnrF_BamHI_R primer (Reverse) | 5′- CCGAGCTCGAATTCGGATCCCAAGCTTGTCGACGGAGCTC -3′ | 34 |

### Example 3: Aloesin production through GtCGT^{V93Q/Y193F}

Aloesin is a representative cosmetic additive extracted from *Aloe vera.* Aloesin is used as a whitening agent in the cosmetics industry due to anti-tyrosinase and anti-melanogenesis effects thereof, and is receiving attention as a potential drug or cosmetic raw material due to antiinflammatory and anti-radical properties thereof. However, aloesin content in plants is very low, requiring a more efficient preparation method. The production of aloesin has been reported in an existing paper (D Yang et al., Proc. Natl. Acad. Sci. U. S. A. 2018, 115 (40), 9835-9844). However, C-glycosyltransferase that converts aloesone into aloesin has not yet been reported (FIG. 8), and thus in the present invention, whether the GtCGT mutant developed in Example 2 is effective at producing aloesin was tested.

In the present invention, in order to produce aloesone, the *E. coli* BL21 (DE3) strain was transformed with the following plasmids: pCDF-RpALS, pWAS-anti-pabA, and pBBR1-zwf. Therefore, the strain expresses the following genes: *RpALS* (encoding *R. palmatum* aloesone synthase), anti-pabA synthesis regulatory sRNA, and zwf (encoding *E. coli* glucose 6-phosphate 1-dehydrogenase) (Yang, D.; Kim, W. J.; Yoo, S. M.; Choi, J. H.; Ha, S. H.; Lee, M. H.; Lee, S. Y., Repurposing type III polyketide synthase as a malonyl-CoA biosensor for metabolic engineering in bacteria. Proc. Natl. Acad. Sci. U. S. A. 2018, 115 (40), 9835-9844).

The strain produced 30.9 mg/L of aloesone from glucose. Before production of aloesin, *RpALS* was additionally introduced into a compatible plasmid in order to increase aloesone production. To this end, RpALS was introduced into the pRSFDuet-1 plasmid with a high copy number RSF origin of replication. RpALS was amplified from the previously constructed pCDF-RpALS using ALS_NdeI_F and ALS_NdeI_R primers, and then inserted into the NdeI site of pRSFDuet-1 using Gibson assembly to construct a relevant plasmid. Thereafter, pCDF-RpALS and pRSF-RpALS were simultaneously transformed into the E. *coli* BL21(DE3) strain already containing the pWAS-anti-pabA and pBBR1-zwf plasmids, and flask culture of the strain was performed. Thereby, 102.1 mg/L of aloesone was produced, confirming greatly increased aloesone production compared to before improvement.

**[Table 9]**

| Name | Sequence | SEQ ID NO: |
|---|---|---|
| ALS_NdeI_F primer (Forward) | 5′- GTATAAGAAGGAGATATACATATGGCAGATGTCCTGCAGGAG -3′ | 35 |
| ALS_NdeI_R primer (Reverse) | 5′- GATATCCAATTGAGATCTGCCAATTAGGGAATGGGGACGCTAC - 3′ | 36 |

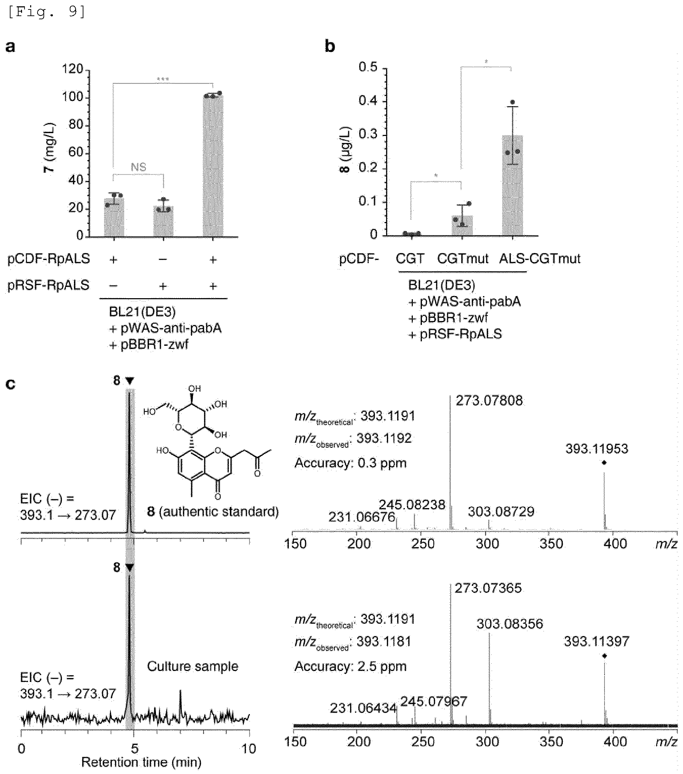

Thereafter, in order to test the production of aloesin, the pCDF-GtCGT or pCDF-GtCGT^{V93Q/Y193F} plasmid was transformed into the BL21(DE3) strain containing pWAS-anti-pabA, pRSF-RpALS, and pBBR1-zwf plasmids, followed by flask culture. Thereby, the strain containing GtCGT^{V93Q/Y193F} produced 0.06 µg/L of aloesin, and succeeded in producing a greater amount of aloesin than the strain containing GtCGT (FIG. 9).

Similarly to the test for increased production of aloesone, RpALS was additionally introduced to increase aloesin production. To this end, pCDF-RpALS-GtCGT^{V93Q/Y193F} was constructed instead of introducing pCDF-GtCG^{TV93Q/Y193F}. Specifically, RpALS was amplified from pCDF-RpALS using pCDFDuet_F and pCDFDuet_R primers, and inserted into the NcoI and BamHI sites of the pCA plasmid through Gibson assembly. Thereafter, four plasmids, namely pRSF-RpALS, pCDF-RpALS-GtCGT^{V93Q/Y193}, pWAS-anti-pabA, and pBBR1-zwf, were transformed into *E. coli* BL21(DE3), which was named an ALS strain.

The ALS strain succeeded in producing 0.3 µg/L of aloesin from glucose. The produced aloesin was identified through LC-MS/MS as shown in FIG. 9.

As such, the present inventors succeeded in producing aloesin in a state where the enzyme was not known through introduction of GtCGT, and were able to greatly increase aloesin production capacity through introduction of GtCGT^{V93Q/Y193F}. Here, the GtCGT^{V93Q/Y193F} enzyme mutant can be said to be an enzyme capable of producing glycosides of polyketides, and the present inventors proceeded to create additional mutants based on GtCGT^{V93Q/Y193F} in order to construct a mutant capable of further increasing aloesin production capacity.

### Example 4: Increased production of aloesin through introduction of additional mutant into GtCGT^{V93Q/Y193F}

The present inventors attempted to increase the conversion efficiency from aloesone to aloesin by further improving the above-developed GtCGT^{V93Q/Y193F} mutant showing activity on aromatic polyketides. To this end, aloesone, which is a new substrate, was docked to the GtCGT^{V93Q/Y193F} structural model produced in Example 2. Thereby, mutants expected to form a more stable bond with aloesone and increase enzymatic activity were selected as shown in Table 10 below.

**[Table 10]**

| No. | Mutant |
|---|---|
| 1 | I18P |
| 2 | Q20M |
| 3 | T50N |
| 4 | T50Q |
| 5 | T50K |
| 6 | T50R |
| 7 | T50V |
| 8 | I95M |
| 9 | I95T |
| 10 | V290G |
| 11 | V290A |
| 12 | I323S |
| 13 | I323A |
| 14 | I95L |

Mutagenesis for a specific sequence was performed in the same manner as in Example 2. Here, plasmids containing gene mutants were constructed using pCDF-RpALS-GtCGT^{V93Q/Y193F} as a template and using primer pairs shown in Table 11 below. The plasmids thus constructed were transformed into the BL21 (DE3) strain along with three plasmids, namely pWAS-anti-pabA, pRSF-RpALS, and pBBR1-zwf, followed by flask culture under the same conditions as in Example 3 using the strains thus constructed. The concentration of aloesin produced was measured in an MRM mode of the HPLC Triple Quadrupole Mass Spectrometer (LCMS-8050, Shimadzu) of the KAIST Biocore Center.

**[Table 11]**

| Name | Sequence (5'-3') | SEQ ID NO: |
|---|---|---|
| GtCGT ^{V93Q/Y193F}_I18P_F | tgtgcttcCCAggccagggcgtggtcaat | 63 |
| GtCGT ^{V93Q/Y193F}_I18P_R | cgccctggccTGGgaagcacacaagaaaaat | 64 |
| GtCGT ^{V93Q/Y193F}_Q20M_F | ttcatcggcATgggcgtggtcaatcccat | 65 |
| GtCGT ^{V93Q/Y193F}_Q20M_R | tgaccacgcccATgccgatgaagcacacaag | 66 |
| GtCGT ^{V93Q/Y193F}_T50K_F | aatcgttggaaAGgagatccgtaaggcgaa | 67 |
| GtCGT ^{V93Q/Y193F}_T50K_R | tacggatctcCTttccaacgatttccggtg | 68 |
| GtCGT ^{V93Q/Y193F}_T50N_F | aatcgttggaaAtgagatccgtaaggcgaa | 69 |
| GtCGT ^{V93Q/Y193F}_T50N_R | tacggatctcaTttccaacgatttccggtg | 70 |
| GtCGT ^{V93Q/Y193F}_T50Q_F | aatcgttggaCAGgagatccgtaaggcgaa | 71 |
| GtCGT ^{V930/Y193F}_T50Q_R | tacggatctcCTGtccaacgatttccggtg | 72 |
| GtCGT ^{V93Q/Y193F}_T50R_F | aatcgttggaCGtgagatccgtaaggcgaa | 73 |
| GtCGT ^{V93Q/Y193F}_T50R_R | tacggatctcaCGtccaacgatttccggtg | 74 |
| GtCGT ^{V93Q/Y193F}_T50V_F | aatcgttggaGTtgagatccgtaaggcgaa | 75 |
| GtCGT ^{V93Q/Y193F}_T50V_R | tacggatctcaACtccaacgatttccggtg | 76 |
| GtCGT ^{V93Q/Y193F}_I95L_F | gtatggcaatacCtcaatcacttagaccagacag | 77 |
| GtCGT ^{V93Q/Y193F}_I95L_R | ggtctaagtgattgaGgtattgccatacgtcaaacg | 78 |
| GtCGT ^{V93Q/Y193F}_I95M_F | gtatggcaatacatGaatcacttagaccagacag | 79 |
| GtCGT ^{V93Q/Y193F}_I95M_R | ggtctaagtgattCatgtattgccatacgtcaaacg | 80 |
| GtCGT ^{V93Q/Y193F}_I95T_F | gtatggcaatacaCTaatcacttagaccagacag | 81 |
| GtCGT ^{V93Q/Y193F}_I95T_R | ggtctaagtgattAGtgtattgccatacgtcaaacg | 82 |
| GtCGT ^{V93Q/Y193F}_V290A_F | gtgtcgggtctgCtgtatatttgaagcaggag | 83 |
| GtCGT ^{V93Q/Y193F}_V290A_R | gcttcaaatatacaGcagacccgacactaacg | 84 |
| GtCGT ^{V93Q/Y193F}_V290G_F | gtgtcgggtctgGtgtatatttgaagcaggag | 85 |
| GtCGT ^{V93Q/Y193F}_V290G_R | gcttcaaatatacaCcagacccgacactaacg | 86 |
| GtCGT ^{V93Q/Y193F}_I323A_F | agcaaacgcGCcggtacggaaccgcatgt | 87 |
| GtCGT ^{V93Q/Y193F}_I323A_R | gttccgtaccgGCgcgtttgctcgg | 88 |
| GtCGT ^{V93Q/Y193F}_I323S_F | agcaaacgcaGcggtacggaaccgcatgt | 89 |
| GtCGT ^{V93Q/Y193F}_I323S_R | gttccgtaccgCtgcgtttgctcgg | 90 |

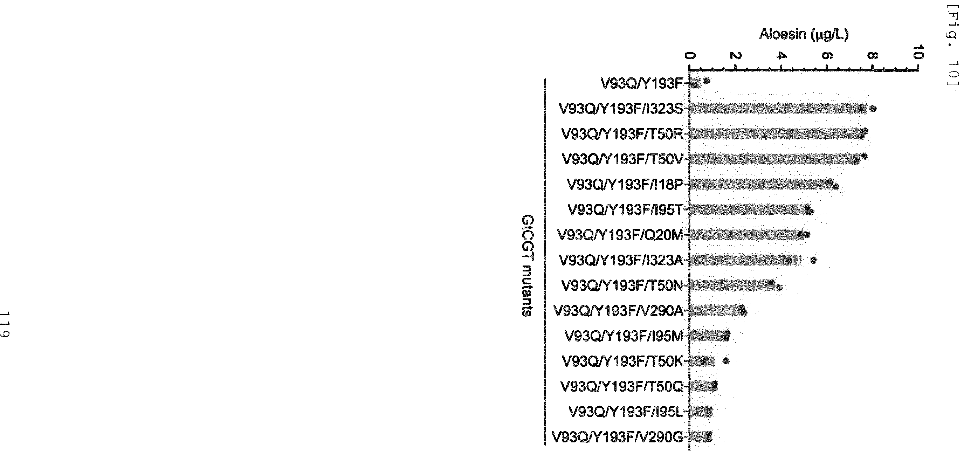

Based on results of flask culture, introduction of additional mutants such as I323S, T50R, T50V, I18P, I95T, Q20M, and I323A increased the aloesin production 10-fold or more, and in particular, 7.75 µg/L of aloesin was produced through the GtCGT V93Q/Y193F/I323S mutant (FIG. 10), which is a 25.8-fold or more increase compared to the conventional concentration of 0.3 µg/L.

In addition to searching for additional mutants based on structures, computer-based docking simulation (SW: AutoDock Vina) using aloesone as a ligand was performed using the GtCGT structural model in order to further increase enzymatic activity. Thereby, 15 mutants showed higher docking scores than the wild-type enzyme (Table 12).

**[Table 12]**

| No. | Mutant |
|---|---|
| 1 | V22A |
| 2 | L29A |
| 3 | E46G |
| 4 | V48G |
| 5 | E51C |
| 6 | A55S |
| 7 | S86V |
| 8 | D99G |
| 9 | R103V |
| 10 | C151G |
| 11 | L184G |
| 12 | L194A |
| 13 | E332P |
| 14 | I18A |
| 15 | P385A |

Mutagenesis for a specific sequence was performed in the same manner as in Example 2. Here, plasmids containing gene mutants were constructed using pCDF-RpALS-GtCGT^{V93Q/Y193F} as a template and using primer pairs shown in Table 13 below. The plasmids thus constructed were transformed into the BL21 (DE3) strain along with three plasmids, namely pWAS-anti-pabA, pRSF-RpALS, and pBBR1-zwf, followed by flask culture under the same conditions as in Example 3 using the strains thus constructed. The concentration of aloesin produced was measured in an MRM mode of the HPLC Triple Quadrupole Mass Spectrometer (LCMS-8050, Shimadzu) of the KAIST Biocore Center.

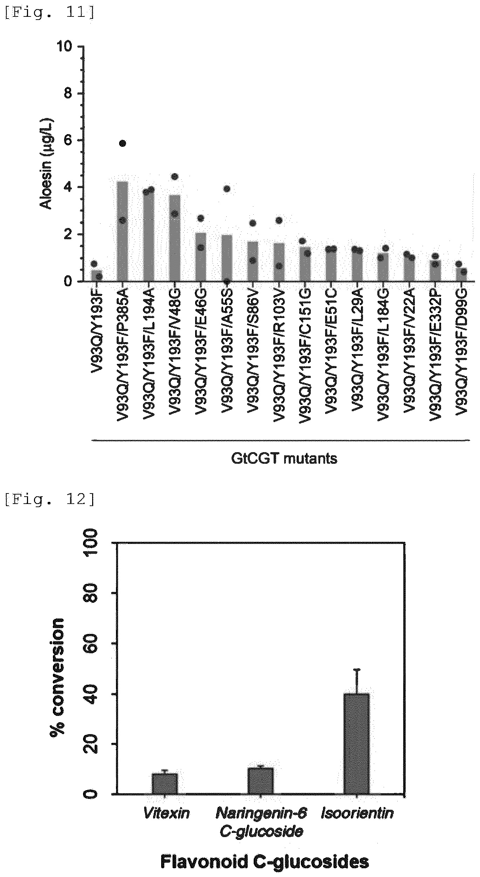

Based on results of flask culture, introduction of additional mutants, such as P385A, L194A, and V48G increased the aloesin production 5-fold or more, and in particular, 4.23 µg/L of aloesin was produced through the GtCGT V93Q/Y193F/P385A mutant (FIG. 11), which is a 14.1-fold or more increase compared to the conventional concentration of 0.3 µg/L.

**[Table 13]**

| Name | Sequence (5'-3') | SEQ ID NO: |
|---|---|---|
| V22A-f | cggccagggcgCggtcaatcccatgttacg | 91 |
| V22A-r | tgggattgaccGcgccctggccgatgaagc | 92 |
| L29A-f | catgttacgtGCggggaaggcgttcgcctc | 93 |
| L29A-r | cgccttccccGCacgtaacatgggattgac | 94 |
| E46G-f | agcgcaccggGCatcgttggaactgagatc | 95 |
| E46G-r | tccaacgatGCccggtgcgcttaaagtgac | 96 |
| V48G-f | ccggaaatcgGtggaactgagatccgtaag | 97 |
| V48G-r | ctcagttccaCcgatttccggtgcgcttaa | 98 |
| E51C-f | cgttggaactTGCatccgtaaggcgaataac | 99 |
| E51C-r | ccttacggatGCAagttccaacgatttccgg | 100 |
| A55S-f | gatccgtaagTcgaataaccttaatgatgaccaac | 101 |
| A55S-r | aaggttattcgActtacggatctcagttcc | 102 |
| S86V-f | cgtaaacggtGTcaaaccgtttgacgtatgg | 103 |
| S86V-r | caaacggtttgACaccgtttacggattccca | 104 |
| D99G-f | caatcacttagGccagacaggccgtcaaaaac | 105 |
| D99G-r | ggcctgtctggCctaagtgattgatgtattgc | 106 |
| R103V-f | ccagacaggcGTtcaaaaacttccgattatg | 107 |
| R103V-r | ggaagtttttgaACgcctgtctggtctaagtg | 108 |
| C151G-f | ggtccaatctGgtgcaagtttttcagcatat | 109 |
| C151G-r | gaaaaacttgcacCagattggacccacaagg | 110 |
| L184G-f | ctgggatgccaGGtttgaaatatgatgaagtg | 111 |
| L184G-r | catatttcaaaCCtggcatcccaggaagttg | 112 |
| L194A-f | cccgacttcGCgcatccgcgcacaccctac | 113 |
| L194A-r | gtgcgcggatgcGCgaagtcgggcacttcatc | 114 |
| E332P-f | tgtactgcccCCggagttctgggagaaggcc | 115 |
| E332P-r | cccagaactccGGgggcagtacatgcggttc | 116 |
| I18A-f | tgtgtgcttcGCcggccagggcgtggtcaat | 117 |
| I18A-r | cgccctggccgGCgaagcacacaagaaaaat | 118 |
| P385A-1 | catcactttcGcacaatttggggaccaagtg | 119 |
| P385A-r | ccccaaattgtgCgaaagtgatgacgggcac | 120 |

### Example 5: Production of phenylpropanoid glycoside through GtCGT^{V93Q/Y193F}

In order to test applicability of the C-glycosyltransferase developed in the present invention to phenylpropanoid-based natural products, the present inventors conducted the following experiment.

In order to confirm the enzymatic activity of intracellularly expressed GtCGT^{V93Q/Y193F}, strains in which both pCDF-GtCGT^{V93Q/Y193F} and pBBR1-galU-pgm-ndk were transformed into *E. coli* BL21 (DE3) were subjected to flask culture, and when cell growth reached OD600 of 0.6-0.8, 1 mM IPTG was administered. As such, 70 µM luteolin, 0.5 mM naringenin, or 185.2 µM apigenin was co-administered, followed by culture for an additional 36 hours. The amounts of substrate and product were analyzed by LC-MS. Flask culture was carried out in a 250 mL baffled flask containing 50 mL of R/2 medium (supplemented with 3 g/L yeast extract and 20 g/L glucose), and culture was performed at 30°C and 200 rpm.

Based on results of culture, 15.0 µM vitexin was produced from 185.2 µM apigenin, 51.6 µM naringenin-6-C-glucoside was produced from 0.5 mM naringenin, and 27.9 µM isoorientin was produced from 70 µM luteolin. These values correspond to conversion rates of 8.1%, 10.3%, and 27.9%, respectively (FIG. 10).

As described above, the glucosyltransferase of the present invention also shows activity of producing various phenylpropanoid C-glucosides, indicating that the enzyme of the present invention is a universal enzyme capable of producing various polyketide and phenylpropanoid C-glucosides.

### Example 6: Purification of GtCGT^{V93Q/Y193F} and measurement of K_{M} and Vₘₐₓ

In order to investigate the characteristics of the C-glycosyltransferase GtCGT^{V93Q/Y193F} developed in the present invention in more detail, the enzyme was purified and enzyme kinetic parameters were measured. For enzyme purification using His-tag, pCDF-NHis-GtCGT and pCDF-NHis-GtCGTmut plasmids expressing GtCGT and GtCGT^{V93Q/Y193F} with 6xHis-tag linked to the N-terminus were constructed. Each plasmid was constructed by PCR amplification of pCDF-GtCGT and pCDF-GtCGTmut using GtCGT_N_His_IV_F and GtCGT_N_His_IV_R primers, followed by blunt-end ligation through DpnI treatment and T4 PNK and T4 ligase treatment. Each of the two plasmids thus constructed was transformed into the *E. coli* BL21 (DE3) strain, followed by seed culture in a test tube containing 10 mL of LB and then culture at 37°C until OD₆₀₀ reached 0.8 in a flask containing 500 mL of LB. After treatment with 1 mM IPTG for enzyme expression, additional culture was carried out at 20°C for 16 hours, and cells were collected by centrifugation and then resuspended in 30 mL of lysis buffer (50 mM NaH₂PO₄, 0.3 M NaCl, 10 mM imidazole, pH 7.5). The cells were disrupted by ultrasonication and then centrifuged at 10,000 rpm and 4°C for 40 minutes to obtain a supernatant containing water-soluble proteins. The supernatant was allowed to flow through TALON resin (Clontech) to purify only proteins with His-tag bound thereto. After removal of impurities using wash buffer (50 mM NaH₂PO₄, 0.3 M NaCl, 20 mM imidazole, pH 7.5), the enzyme was purified using elution buffer with 90, 160, 230, or 300 mM imidazole added to the lysis buffer.

**[Table 14]**

| Name | Sequence (5'-3') | SEQ ID NO: |
|---|---|---|
| GtCGT_N_His_IV_F | CACCATCACCATCACCATGGGAGTTTGACTAACAACGATAATC | 121 |
| GtCGT_N_His_IV_R | CATATGTATATCTCCTTCTTATAC | 122 |

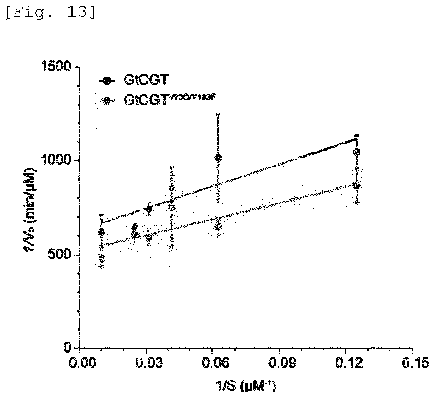

The purified enzyme was buffer-exchanged with an enzyme storage solution (50 mM HEPES, 20% glycerol, pH 7.5) using Amicon Ultra-15 Centrifugal Filters (regenerated cellulose, 50,000 NMWL; Merck), and in order to calculate K_{M} and Vₘₐₓ values, FK was converted into dcII using the purified enzyme. Here, a UDP-Glo Glycosyltransferase Assay Kit (Promega) was used to determine the extent of reaction. Since this kit enables measurement of free UDP generated as a reaction byproduct by luminescence, 200 µL of an enzyme reaction solution containing 0.1 µM enzyme and various concentrations of FK (50 mM HEPES, 0.1 mM UDP-glucose, 5 mM MgCl₂, pH 7.5) was allowed to react at 25°C for 1 hour, 25 µL thereof was taken out, and the amount of emitted light was measured using the kit. The K_{M} and Vₘₐₓ values of GtCGT and GtCGT^{V93Q/Y193F} were calculated by substituting the reaction rate and the substrate concentration into the Michaelis-Menten equation and then performing analysis using OriginPro 2019 software. Consequently, the K_{M} value of GtCGT^{V93Q/Y193F} was decreased by 19.5% compared to GtCGT, whereas the Vₘₐₓ value of GtCGT^{V93Q/Y193F} was increased by 18.2% compared to GtCGT (FIG. 13; Table 15) . Therefore, the Vₘₐₓ/K_{M} value of GtCGT^{V93Q/Y193F} was increased by 46.8% compared to GtCGT, indicating increased catalytic efficiency of the GtCGT^{V93Q/Y193F} mutant.

**[Table 15]**

| | *K*_{M} (µM) ^{a} | *V*ₘₐₓ (µM min⁻¹) ^{a} | *V*ₘₐₓ/*K*_{M} (min⁻¹) |
|---|---|---|---|
| DnrF | 56.75 ± 4.68 | 0.99 ± 0.086 | 1.75 × 10⁻² |
| DnrF^{P217K} | 43.31 ± 2.64 | 1.01 ± 0.002 | 2.34 × 10⁻² |
| GtCGT | 6.30 ± 1.64 | 1.60 × 10⁻³ ± 1.39 × 10⁻⁴ | 2.54 × 10⁻⁴ |
| GtCGT^{V93Q/Y193F} | 5.07 ± 0.74 | 1.89 × 10⁻³ ± 1.13 × 10⁻⁴ | 3.73 × 10⁻⁴ |
| ^{a} means ± standard deviation (SD: *n* = 3) | | | |

### Example 7. Genetic information

**1. *ravC* from *Streptomyces ravidus* (SEQ ID NO: 37)** 
atgtccagtttcagtattgatgatctgaagcgtatcttgcgcgaaggggcaggggcaacggct

gagttagacggtgacattttagacgcctcctttgatgatttggggtatgattctttggctctt

cttgaaacgggttcgcgcatcggacgtgaatacggtttggaatttgaggatacagctttcgcc

gacgtggaaacacctcgtgacttggtcggcgtagttaatgctcagttatcggccccggctccg

cgtgggtaa
**2. *zhuIJ* from *Streptomyces sp.* R1128 (SEQ ID NO: 38)** atgcgtcatgtagagcatacagtcaccgttgcggccccagcagacttggtttgggaggtactt 
gccgatgtcttaggctatgctgacatcttcccaccgacggaaaaagttgaaattcttgaggag

gggcaaggataccaggtagtgcgccttcacgtcgatgttgcgggtgagattaatacatggacc

agtcgtcgcgatttagaccctgcgcgccgcgtaattgcttaccgccaacttgagacggctccg

atcgtgggccacatgagcggggaatggcgtgctttcacactggatgccgaacgtacccaatta

gtcctgactcacgatttcgtaacccgtgcagccggggatgacggtttagtcgccggaaaattg

accccagatgaggcgcgcgaaatgttagaagcggtggtagaacgtaactctgtcgccgactta

aacgcggtccttggagaagctgagcgtcgcgtccgcgcagccggtggagttggtaccgtaact

gcgtaataataattttgtttaactttaagaaggagatatatccatgtcagggcgcaaaacctt

tttagacttaagttttgctacccgcgacacaccgtcggaggcgactccggtggtggtagattt

gctggaccacgtaactggagccaccgtattaggattatcacctgaggatttccccgatggtat

ggctatttccaatgagaccgttacgttgacgacccacactggcacgcacatggatgcgccact

gcactatggtcccttaagtgggggagttccggcaaagtcgattgaccaagtgcccttggaatg

gtgctatggacctggagttcgtttggatgttcgccacgtgccggcaggagatggtattactgt

cgatcatttgaacgccgcgttggatgcagcagagcacgatttggcccccggtgacattgtgat

gctgtggaccggcgcggacgctctgtggggaacccgcgaatacttgagcacgtttccggggtt

aactgggaaggggacacaatttttggtcgaggcgggtgttaaagtcattggcattgatgcatg

gggactggatcgcccgatggcagctatgatcgaagaataccgtcgtacgggcgataaaggagc

attatggccggctcacgtctatggacgcacacgcgaatacctgcaattagagaagcttaataa

tttgggcgctttaccaggagctacagggtatgacatttcatgctttccggttgcggttgcagg

cactggagctgggtggactcgtgtggtcgccgttttcgagcaagaggaagaggattaataa
**3. *trTT7* from *Arabidopsis thaliana* (SEQ ID NO: 39)** 
atgtcccatcgccgcaaccgtagccacaacaatcgtttacccccgggacctaatccctggccc

attatcggaaacctgccgcacatgggtacgaagccccaccgtacgttgtcggctatggttacg

acctatggcccaattctgcacctgcgtctgggttttgtagacgtggtcgtagcggcgtccaag

tcggtcgccgagcagttcttgaagattcacgacgctaacttcgcttcacgtccccccaactca

ggagccaaacacatggcgtataattatcaagacttggtattcgccccctatggacatcgctgg

cgcttattgcgcaagatcagctcggtgcatctgttctcggcaaaagcgttggaggactttaaa
 
cacgtgcgtcaggaagaggtcggaacactgactcgcgaattagtacgtgtcggcaccaaacca
gtgaaccttgggcaactggtgaatatgtgcgttgtcaacgccttaggacgcgaaatgatcgga
cgccgcttattcggggccgatgccgatcataaggcggatgagttccgctcgatggtcacggag
atgatggcgttagcgggggtctttaatatcggcgactttgtaccgtcattagactggcttgat
ctgcaaggggtcgctggaaagatgaagcgtttacataagcgttttgatgcgttcttaagttcg
attttaaaagaacatgaaatgaatgggcaagaccaaaagcataccgatatgttatcgacctta
atcagccttaagggtacagatctggatggggatggcggctccttaacggatactgaaattaag
gcgcttttattaaacatgttcacagccggaaccgacacatcagccagtacagtagattgggca
atcgctgaattgatccgccaccccgatatcatggtgaaggctcaggaagaattagatattgtt
gtaggtcgcgaccgccctgtgaatgagtctgatatcgcccaactgccgtacttacaggcggta
attaaggaaaattttcgtctgcatccacctactcccctgtctttgccgcacattgcgagtgaa
tcctgtgagattaacggttaccatattcccaaaggttcaacattacttaccaacatctgggct
atcgcccgtgatccggaccagtggagcgatccgttagcttttaaaccagaacgttttctgcca
ggaggagaaaaatctggggttgatgtaaaaggtagtgatttcgagctgattccgttcggtgca
ggccgtcgcatttgtgcaggcctgtctctgggtcttcgcacgatccagttcttaacagcgact
ttagtacaagggtttgattgggagttagctgggggagtgacgcccgaaaaactgaacatggaa
gaatcgtacgggttaactttgcaacgcgctgtccctctggtagtacacccgaaacctcgtttg
gccccgaatgtgtacgggctgggcagtggctaa
**4. *ATR2* from *Arabidopsis thaliana* (SEQ ID NO: 40)** 
atgtcttcttcttcttcttctacctctatgatcgacctgatggctgctatcatcaaaggt

gaaccggttatcgtttctgacccggctaacgcttctgcttacgaatctgttgctgctgaactg

tcttctatgttaattgagaatcgtcagtttgctatgatcgttacaacatccatcgcggtcctt

attggttgtatcgttatgttggtctggcgccgctctggttccggtaactctaaacgtgtggaa

ccgcttaaaccgctggtgatcaaacctcgtgaggaggaaatcgacgatggacgtaaaaaagta

acaatctttttcggaacgcagactggcactgcggaaggttttgccaaggcattaggtgaggaa

gctaaagctcgttatgaaaagacgcgcttcaagattgttgatctggacgattacgctgcagac

gatgatgaatacgaggaaaaattaaaaaaagaggatgtagctttcttcttcttagctacgtat
 
ggcgatggtgaaccgacagataatgccgctcgtttttataagtggtttaccgaaggcaatgat

cgtggtgagtggttgaaaaacttaaaatatggggttttcgggctgggcaatcgtcaatacgag

cactttaacaaggtcgcgaaagtggtcgatgacattctggttgagcaaggcgcacagcgtctg

gtacaagtagggttaggggatgatgaccagtgtatcgaagatgatttcacagcttggcgcgaa

gcattgtggcccgagttggatacgattctgcgcgaagagggcgatacggctgttgccacaccc

tacacagccgcagtattagagtatcgcgtaagcatccatgatagcgaggatgccaaattcaat

gatattaaccttgctaacggaaacgggtatacagtttttgacgctcaacatccgtataaggcc

aacgttgcggtcaaacgtgaattgcacaccccggagtccgaccgttcctgtatccatctggaa

tttgatattgcgggatcaggtttaacatacgaaactggagatcacgttggtgttctgtgcgat

aacttatccgagacggtggatgaggcactgcgccttttagacatgtcccctgacacgtatttt

agcttgcatgctgaaaaagaggacggtactccgatcagtagctcgctgccaccgccgtttcca

ccgtgcaatttacgcacggctttaacacgttacgcgtgcctgttgtcatctcctaagaaatcc

gccttagtggctttggctgcacacgctagtgatcccactgaggccgagcgcttgaaacactta

gcaagccctgcaggtaaagacgagtactccaagtgggtagtagagtcacagcgtagtttattg

gaggtgatggccgagtttcctagtgcgaagccaccgttgggagttttctttgccggggtggct

ccgcgtttgcaaccacgtttttatagcatcagtagttctccaaaaatcgccgagactcgcatt

cacgttacatgtgccctggtctacgaaaaaatgccgactgggcgcatccacaagggtgtatgc

tcgacttggatgaagaacgccgtaccctacgaaaagtctgaaaactgcagctcggcgccaatc

ttcgtacgccagtccaatttcaagttgccgtcagattcaaaggtaccgatcattatgatcggt

ccaggaacggggttagctccgttccgtgggttcttacaggaacgcttagcactggtcgagtcg

ggggtagaattgggcccctccgtcttgtttttcgggtgtcgtaaccgtcgcatggacttcatc

tatgaagaagagctgcaacgtttcgtggaaagtggggcgcttgctgaactgtcggtggcgttt

tcccgcgaaggacccacgaaagaatatgttcaacacaaaatgatggacaaagcgtcggatatc

tggaacatgatttcacagggcgcttatttatatgtatgtggcgatgcgaaaggcatggcgcgt

gacgtccaccgttctctgcacaccattgcgcaagagcaaggtagcatggattcaacgaaagca

gaaggcttcgtgaagaatttacaaacctctgggcgctatcttcgtgatgtgtggtaa
**5. *UrdGT2 (SfCGT)* from *Streptomyces fradiae* TU2717 (SEQ ID NO: 41)** 
atgtttgccctggctccgctggccacagcagctcgtaatgcaggtcatcaggtagtaatggca

gcaaaccaggacatgggacctgtcgtaaccggggttggccttccagccgtagcaaccactgat

cttccgatccgtcatttcatcactaccgatcgtgaaggacgtcccgaggccattccttctgac

ccggtcgcgcaggcccgtttcactggtcgctggttcgcccgtatggctgccagttccttgccc

cgtatgcttgatttttcacgtgcatggcgcccagacttaatcgtcggtggtactatgagctat

gtcgctccgctgttagctcttcacctgggagtcccgcacgcccgtcagacttgggatgcggta

gacgctgatggaatccacccaggtgctgatgctgagcttcgcccagagttaagcgaattggga

ttggagcgccttcccgcacccgatttgttcatcgacatttgcccgccctcgttacgtcctgcc

aacgcagcaccagctcgcatgatgcgccacgtagccacgagccgccaatgcccgttagagccg

tggatgtatacacgtgacactcgccagcgtgttttagtgacgtcgggatcgcgtgttgcaaaa

gaatcttacgatcgtaatttcgattttttacgtggattagcgaaggatttggtgcgctgggat

gttgaattaattgtggctgctcctgacaccgtggctgaggctcttcgtgccgaggtgccacaa

gctcgcgtagggtggacccctttagacgtcgtggcccctacatgcgatttattggtgcatcac

gccggcggagtctctacgctgactggtttatcggctggcgagccccaattattaatcccaaag

ggcagtgtattggaagctcctgcgcgccgcgtagcagattacggcgcggcgattgcactgttg

cctggtgaggactcgacggaagctatcgccgatagttgtcaggagttgcacgccaaggacact

tatgcccgccgcgctcaagacttaagccgcgaaatttcagggatgcctctgccggccacagtg

gtgactgcactggaacagttagcctaa
**6. *DcUGT2 (DcCGT)* from *Dactylopius coccus* (SEQ ID NO: 42)** 
atggagttccgcttattgattctggcactgtttagtgtcttaatgagtacgtcaaatggtgcc

gagattcttgccctgtttccaattcacggaatttccaactacaacgtagcagaggcacttctt

aaaacgcttgccaaccgtggacataacgtcactgtggtcacgtcattcccccagaagaaaccc

gtgccaaacttgtatgaaatcgacgtatctggggccaaaggtcttgctaccaactcaattcac

tttgagcgtcttcagactatcattcaagacgtgaagtccaacttcaagaatatggtgcgtctt
 
tcacgcacttactgcgagattatgttctccgatcctcgcgtacttaacatccgcgacaaaaag
tttgatttggtaatcaacgcggtgttcggatcggattgtgacgctggctttgcgtggaaaagc
caagcacccctgatctcaattttaaatgcacgccacaccccgtgggcgcttcaccgtatggga
aacccttctaaccctgcgtacatgccagtaatccactcacgcttcccagtaaaaatgaatttc
ttccagcgtatgatcaatacaggctggcatttatactttttgtatatgtacttttattatggc
aacggagaagacgcaaataaaatggcgcgtaagtttttcggtaacgacatgcccgacattaac
gaaatggtctttaacacatcgttgctttttgttaatactcactttagtgttgatatgccctac
cctcttgttcctaactgcattgaaattggtgggattcacgtcaaagagccccaaccgcttccc
ctggagattcaaaaattcatggatgaagcagagcatggtgtaatctttttcactttgggctcg
atggtccgcactagcacctttcccaatcagactatccaagcgtttaaagaggcgttcgcagaa
cttccacagcgtgttctttggaagtttgaaaacgagaatgaagacatgccttctaacgtttta
atccgcaaatggtttccacagaacgatatctttggacataagaacattaaggcgtttatctcg
catggcggtaactcaggggcccttgaagccgtgcatttcggcgtgcccatcatcggcattcct
ctgttctatgatcagtatcgtaatatcttgagcttcgtgaaagaaggggttgcggtactgctg
gacgttaatgatttgacaaaggataacattctgtcgtctgttcgtacagtagtgaacgataaa
tcttacagcgaacgcatgaaagctctgtcccagctgtttcgcgatcgtccaatgagtcccctg
gacacggctgtctattggacggaatatgtaatccgtcaccgcggagcacatcatctgaagact
gctggcgcttttttgcactggtatcagtatttgttgctggatgtgattactttccttttggta
acattctgtgccttttgcttcattgtcaagtacatctgtaaggcgctgattcaccattattgg
tcgagttccaagagtgaaaaattaaagaaaaactaa
**7. *UGT708A6 (ZmCGT)* from *Zea mays* (SEQ ID NO: 43)** 
atggctgctaatgggggggatcatacctccgcgcgcccacatgtggtgttgcttccatccgct

ggcatgggacatcttgtccctttcgcccgcttagctgtggctttatctgagggacacggctgc

aacgtaagtgtagctgcagttcaaccaacggtttcctctgcggagtcgcgtctgttagacgca

cttttcgtcgccgccgccccagccgtccgccgtcttgatttccgcctggcccccttcgatgaa

tccgagttccccggtgcagacccttttttcttacgcttcgaggcgacacgtcgctcggcaccg

cttctggggccgttattagatgcggcggaagcctccgcacttgtgactgatattgtccttgct
 
tcggtagcgttgccagtggcgcgcgagcgtggagttccctgctatgtgctttttacgtcatcg

gccgcaatgctgtcgttgtgtgcgtattttccagcttatttagatgcacatgcagcggccggc

tcggtcggggtcggagtagggaacgtcgacattccaggggtatttcgcatccctaagtcgagc

gtcccgcaagcacttcacgatccagatcatttatttacccagcagtttgtcgcaaatggccgt

tgtttagttgcctgcgacggcattcttgttaacaccttcgacgccttcgagcccgatgcagta

accgcactgcgccaagggtcgatcacagtctctggcggttttccaccagttttcaccgtgggc

ccaatgcttcccgttcgcttccaggcagaggagacggctgactacatgcgttggttgtctgca

caaccaccccgcagtgtcgtctatgtctcgtttggaagtcgcaaggcgattcctcgcgaccag

ttacgtgaattggccgcagggttagaggctagtggcaagcgttttctgtgggtagtaaagtcg

accatcgtcgaccgcgatgataccgccgatctgggcggcttgttgggggacggctttcttgag

cgcgtccaaggtcgtgcatttgtgactatgggatgggtggaacaggaagagattttgcaacat

ggctcggttggcttgtttatctcgcattgtgggtggaatagccttaccgaagccgccgcgttc

ggggtaccagttcttgcctggcctcgtttcggagatcagcgtgtgaacgccgccttagttgcg

cgctctggattgggagcgtgggaagaagggtggacttgggatggtgaggagggacttactaca

cgcaaggaagtggcgaaaaagatcaagggcatgatggggtacgatgctgtagccgaaaaggcg

gccaaagttggtgacgcagctgcggcagcaattgcaaaatgtggcacgagttatcaatctttg

gaagagtttgtacaacgttgccgcgacgccgagcgtaagtaa
**8. *OsCGT* from *Oryza sativa* (SEQ ID NO: 44)** 
atgccttcctcaggagacgctgccggtcgtcgccctcacgtcgtgctgatcccttcagccgga

atggggcacctggtcccgtttggtcgtctggctgtcgcactttcctctgggcacggatgtgat

gtgtctttagtaacagttcttcctactgtgagtacagcggagtcaaagcatcttgatgcactt

tttgacgcattccccgcagttcgccgtcttgacttcgagttggcgccatttgacgcatcagag

tttcccggtgctgaccctttcttccttcgttttgaggcgatgcgccgttcggctccattgctt

ggccctttgctgacgggcgcgggcgctagcgcactggcgacggacattgctttaacgtctgtc

gtaattccagtagcaaaagagcaagggcttccgtgtcacattttattcactgcgtcggccgca

atgttatcattgtgtgcctacttcccaacttatttggatgccaacgctggcggagggggcggt

gtgggcgacgtggatattcctggagtgtatcgcattccgaaggcatcaattccacaagcctta
 
catgatcccaaccacttgtttactcgtcagtttgtggcgaatggtcgtagtcttacctcggcg
gccggtattctggtgaacactttcgatgcgttagagccggaggcagtagctgcattgcagcaa
ggaaaggtagcctccggctttccaccagtattcgcggtggggccgttgctgcctgcctctaac
caggccaaggatccgcaggcaaattacatggagtggctggacgcccagcccgcccgcagcgta
gtttatgtaagtttcgggagtcgcaaggcgatttcacgtgaacaacttcgcgagctggctgct
ggcttagaggggagcggccaccgttttctgtgggtcgtgaaatccaccgtcgtggatcgtgac
gacgcggccgagctgggagagctgttggacgagggttttttagagcgtgtcgagaagcgtgga
ttggtgacaaaggcatgggtcgaccaggaggaggtactgaaacatgaaagcgtagccctgttt
gtctcacattgcggctggaacagcgtgactgaggcggcggcgagcggtgtgcctgtcctggcc
ttaccccgcttcggggaccaacgtgttaattcaggagtggtggcacgtgcaggattaggagta
tgggcggatacttggtcgtgggagggcgaagcaggcgtgattggtgcggaggaaatctcagag
aaggtcaaagcagctatggccgacgaagctttacgtatgaaagctgcatcccttgcagaggca
gccgccaaggcagtggctggcggtgggagtagtcatcgctgtttagcggaatttgcccgtctg
tgtcaaggtggaacttgccgtactaattaa
**9. *UGT708D1 (GmCGT)* from *Glycine max* (SEQ ID NO: 45)** 
atgagttctagtgaaggagtggtacatgtagcttttcttccaagtgcaggaatgggccacttg

aaccctttccttcgcttggcggaccttcattcgttatggttgtaaagtaacgttaatcacc

ccgaagcctactgtatccctggcagaatcgaatttaatttcacgcttttgttccagctttcca

catcaggttacgcaactggacctgaatttagtcagcgttgatccaacgaccgttgacacaatc

gacccattcttcttacaatttgaaaccatccgccgtagtctgcatcttttacctcccatttta

agtcttcttagcactcctttgtctgccttcatttatgacattactcttatcacgcctttgctt

tctgtaatcgagaagctgtcgtgccccagctacttgtattttacatcttcagcacgtatgttc

tctttcttcgcacgtgtctccgtgttgtccgcatctaatcccgggcagactccctcgtcattt

atcggtgacgatggagttaagatccctgggttcacaagccccatcccacgcagcagtgttccg

cctgcgattcttcaagcgtcctcaaatctttttcagcgcattatgttagaagacagcgcgaac

gttaccaagcttaataatggggtcttcatcaatagctttgaagaactggagggcgaagcttta

gccgctttaaacggggggaaagttcttgaaggtctgccgcccgtgtacggggtgggccccctt
 
atggcgtgtgaatatgagaaaggcgacgaggagggtcaaaagggctgcatgtcttcgatcgtg

aagtggctggatgaacagtcgaagggaagcgtggtatacgtgtccttgggcaatcgtacggaa

acgcgccgtgagcagattaaggatatggcccttggtttgatcgagtgtggctatggattcttg

tgggtcgtcaaactgaagcgcgtcgataaagaagatgaggaaggcttagaagaggtgttaggt

agcgagctgagttccaaggttaaggagaagggtgttgtagttaaggaatttgttgaccaagtc

gaaattttgggccacccaagtgttgggggatttttgtcgcacgggggttggaacagcgtaact

gaaactgtatggaagggagtgccttgtctgtcatggccacagcatagtgatcagaagatgtct

gcggaggtaatccgtatgtccggaatgggtatctggcccgaggagtggggctgggggacgcaa

gatgttgtgaagggagacgaaatcgccaaacgcattaaggaaatgatgtcgaacgaatcgttg

cgcgtaaaggcgggagaattgaaggaagcggcgttaaaggcggcaggggtaggggggagttgt

gaagtgactattaaacgtcagatcgaagagtggaaacgcaatgcccaggctaattaa
**10. *GtUF6CGT1 (GtCGT)* from *Gentiana triflora* (SEQ ID NO: 46)** 
atggggagtttgactaacaacgataatcttcatatttttcttgtgtgcttcatcggccagggc

gtggtcaatcccatgttacgtttggggaaggcgttcgcctccaaagggttacttgtcacttta

agcgcaccggaaatcgttggaactgagatccgtaaggcgaataaccttaatgatgaccaacca

atcaaggtgggttccgggatgattcgtttcgaatttttcgacgatggatgggaatccgtaaac

ggtagcaaaccgtttgacgtatgggtctacatcaatcacttagaccagacaggccgtcaaaaa

cttccgattatgttaaagaaacatgaggagacagggactcctgtatcttgcttgatcctgaat

cccttagtcccttgggtcgcggacgtagccgattcacttcagatcccctgcgctaccttgtgg

gtccaatcttgtgcaagtttttcagcatattaccactaccacgggttagtgcctttccca

accgaatcagagcccgagatcgacgtacaacttcctgggatgccacttttgaaatatgatgaa

gtgcccgactacctgcatccgcgcacaccctaccccttttttggcacgaacattttaggtcaa

ttcaagaatttatccaagaacttctgtatcctgatggataccttctacgagttggaacacgag

atcatcgataatatgtgtaaattgtgtccgattaagccaattggcccgttgtttaagattccg

aaagacccaagctccaacggaatcacgggtaatttcatgaaagtggatgactgcaaggagtgg

ctggacagccgtccaacatcaactgtggtttacgttagtgtcgggtctgttgtatatttgaag
 
caggagcaggttacagaaatggcatacggcattttaaattcggaagtttcgtttttgtgggtg

ctgcgcccgccgagcaaacgcatcggtacggaaccgcatgtactgcccgaggagttctgggag

aaggccggagatcgtggcaaggtggtgcaatggtcaccccaggagcaggtgcttgctcacccc

gccactgtcggttttttaacacactgtggatggaatagcactcaagaggcgatttcgagcgga

gtgcccgtcatcactttcccacaatttggggaccaagtgaccaatgctaagttccttgtggag

gaatttaaggtcggggtccgtttaggccgcggagagttagaaaatcgcatcatcacacgcgac

gaagtagaacgcgctttacgcgagattacttcaggccccaaggctgaagaggtaaaagagaac

gccttaaaatggaagaagaaggcagaagagacagtagctaaaggcggctactccgaacgtaat

cttgtaggcttcattgaagaggtggctcgtaagactggtacaaagtaa
**11. *AvCGT* from Aloe *vera* (SEQ ID NO: 47)** 
atggaggaaatttccagtaaagtggagttcttatcccttaagcccagcatgtcaggaagtccc

cgttacagccccacatttcgtaaaatcggaagcggtcgcaattcccgccgcgactcccgtgct

catgcagggaatttcccctggattcgcaacaatcgtgtttttttttggctgcttttaatcacc

atctgggcgtacatcggctttcacgtccaatctcaatgggcacatggcgaccataaagctgag

ttcgttggatacaagtcggaggtagggaagatgggtgaggacgtcaagtcggtaaatagtacg

actacgttctccattgtacacaagggcaatttaactgttgaaggaaagaaagaccccgattcc

aattttggtatttcactgttgaaaaagggtaaacaggttctttcccgtttaaattcacgcaaa

aagggccatcgttcgcgcaaggtgtcggaaaaactggaagaaacggacgacaatgggacg

ggagaaatggatgaggtccttatccagcgcaaaaacacatcttatggcttaattgtagggcct

tttgccaaactggaagagagtgtgcttgagtggagcccaggcaagcgccgtggtgtctgctat

cgtaagggagaatttgcccgcgcggtgtcttctcagcgctttatgttgatcttccacgaattg

tcaatgactggcgccccattgtccatgttggaattggccacggagatcctgtcttgcggtggg

tctgtgagcgctattgtattatctaagaagggagggttaatgccggaactgaagaagcgtggt

attaaggttttgcaagaccgtgacaaggtgagtttcaaggtcgccacgaaagtagacctgatt

attgcgggatctgctgtatgtagtagctggatcgagccatatctggagtatttccccgctggg

tccggacatattgtctggtggatcatggaaaaccgtcgcgaatacttcgaccgtagcaagcat

cttttaaaccgtgtgaaaattttggcatttcttagcgatagccagtcaaagcagtggctttct
 
tggtgtgaggaagagaagattaaattcctgatccagccaatgttagtgccgttgtcagttaac

gatgagctggccttcgttgccggtattccttgtagcttgaatactccagcattatcagtggag

aaaatgatcgaaaagcgtgatttattacgtcacgcagtccgtaaggaaatggggttgggggac

aatgacatgcttgtgatgagtttaagtagcatcaacccagccaagggtcagcgctttctgctt

gaggcagccttactggtagctgaacacaatgtatcattgaaagatgctaacagttacagtctt

atggaggaggagaagttatccgggaacgcacctcaaaatcaaaccatcatgatcggtcaactg

aatcctggccacgtacttcagatcgccaatgacactaataagcccgtcaatgcgttacagaag

attggcgccacacgtgtctcgtcgaagcgtcgcggcaagctgcatacgaatacagtcacgggc

gtgcttcagaaaagccgcaaacttttgtccgaggcagcaggtatgaaggaggaaaccctgaaa

gtccttgtaggttccgtcggatcgaaatcgaataaggttctgtatgtaaaggcaatcatggaa

tacatcagccaacattctaatttgtctaaggtcgttctttggaccccagccaccacgtctatc

gcagcactgtacgccgccgcggacgtgtacgtcattaacgctcagggacatggagagacattc

ggtcgcgtgacgatcgaggcgatggcctttggcctgccagtgctggggactgacgccggaggg

actaaagaaatcatcgaccaccgtgttacgggacttctgcatcctgtgggtcccgagggcact

gtactgttagcgcaacacattcaatatcttttaaaaaatcccagcgtgcgcaagaaaatgggt

atcaatggtcgccgcaaagtacaagataaatacttaaaacaccagacttacgagtcccttggc

aaagtcatgttcaaatcgatgcgtccccgttaa
**12. *dnrF* from *Streptomyces peucetius* ATCC 29050 (SEQ ID NO: 48)** 
gtggccttgacgaagccggatgtcgatgtcctcgtggtgggcggcggtctcggggggctgtcc

accgccctgttcctcgcccgccggggggcgcgggtcctgctggtggagcggcatgccagcacc

tcggtcctgcccaaggcggcaggccagaacccgcgcaccatggaactgttccgcttcggcggc

gtggccgacgagatcctggccacggacgacatccgcggcgcccagggcgacttcaccatcaag

gtcgtggagcgcgtgggcggtcgcgtcctgcacagcttcgcggagagcttcgaggaactggtc

ggtgcgacggaacagtgcacgcccatgccctgggcgctcgctccccaggaccgggtggagccc

gtcctggtggcccacgccgccaagcacggcgcggagatccggttcgccaccgaactgacctcc
 
ttccaggcgggcgacgacggtgtcacggcccgcctgcgcgacctgggcacgggagcggagagc
accgtgagcgcccgctacctggtcgccgccgacggaccccgcagcgcgatccgggagagcctg
ggcatcacccggcacggtcacggcaccctggcccacttcatgggcgtcatcttcgaggccgac
ctcaccgccgtcgtaccgcccgggtccaccggctggtactacctgcagcacccggacttcacc
ggcacgttcggccccaccgaccggcccaaccggcacaccttctacgtccgctacgaccccgaa
cgcggcgagaggccggaggactacacaccgcagcgctgcaccgagctgatccggctggctgtc
gacgcgcccgggctcgtcccggacatcctcgacatccaggcctgggacatggcggcgtacatc
gccgaccggtggcgcgaagggccggtgctgctggtcggcgatgccgccaaggtcaccccgccc
accgggggcatgggcggcaacaccgccatcggcgacgggttcgacgtggcctggaagctggcc
gccgtgctgcgcggcgaggcgggcgagcggctcctcgacagctacggggcggagcggtcgctc
gtgtcccgcctcgtcgtcgacgagtcactcgccatctacgcccagcgcatggctccccacctg
ctcggcagcgttcccgaggaacgcggtacggcgcaggtcgtcctgggcttccgctaccgctcc
accgccgtcgccgccgaggacgacgaccccgagccgaccgaggatccgcgacgcccgtccggg
cgccccggcttccgcgcaccccacgtctggatcgaacaggacggcacacggcgttccaccgtc
gagttgttcggcgactgctgggtgctcctggccgcaccggagggcggcgcctggggccaggcg
gccgcccgcgccgccgcggatctgggcgtccgcctcgacgtccatctcgtcggccgcgatgtc
gccgccccctccggcgaactgacgcggacctacgggatcggccgggcgggggccagcttggtg
cgcccggacggcgtggtcgcctggcgtacggcagtagcgccgggagcggaggcccaggaccag
ctgagcaccctgctcacccggctgctggcccgctga
**13. *antDEF* from *Photorhabdus luminescens* (SEQ ID NO: 49)**

ATGATAATAAATAACAGAAATGAATCTCAACCACGTAGAGTTGTGGTGACAGGGC
TAGGTGTTGTCGCACCGACAGGTGTTGGCGTTAATGAATTTTGGAACAATATTCATAACGGCA
AATCGGGGGTAAGTGAATATGAGTGGGGAAGAAAAAAATTTGGTTTTAAAAGCGGAGCAATAG
GAAAAGTTCACGGTAACGATAGCGATAGCAAAGAGTTTGTGCTGAAAAGTGAGCGTAAATATC
TTGAGTTTGCGCTAGAAGCCTCTGAGATGGCAATGCAAGATGCAAATTTAAAACCTTCAGACA

TTGATGGCCGGCGTTTTGGCGTTGCGATAGCAACAGCGATTGCCGATGCTGCGGGAATGGAAG
AGTGTTTGCTCAGGATCACCAAAGGGGGCAAAGAGAATATTCATCCTGATTTAATTAAATCAG
AGGATTATGACAGCTTTGATTTCAGCTCTGCCGCCACCTCTGTTGCGAAAAAATATGGCGCAT
CGATGTCCGTCAGTAACATATCAACTGGGTGTGCGGCAGGACTTGATGCATTAGGCATTGCGA
TGGAGCATATCCGTTATGGCAGAGCGGATGTGATGCTGGCTGGCGCCAGTGAAGCGCCGCTTT
GTCCACTTTCTATCGGCTCTTTTGAAGCTTTAGGGGCGCTATCATCAAGAGAATTGGAAAATC
AGCAAGCAGCGACTTGTCCTTTTTCCCTTGAGCGGGATGGATTTGTGATTGCTGAAGGGTGTG
GAATATTAATTTTAGAGTCTTATGAACATGCTAAGCAGCGTGGAGCACATATCTATGCTGAAT
TAGCAGGGTATGCGTCCGTGAATAACGCTTATCATATGACCGACTTGCCTGCGGATGGAATGG
CAATGGCGCGGTGCATTGATATGGCGTTGAAGGATGCCCAGATATCGCCATCAGCGGTCAATT
ATATTAGTGCTCATGGCAGTTCTACGGCTCAAAATGATATTAACGAATCAAATGCGATTAAAT
TTGTTTTGGGAGAAAATGCATTTGATATTCCAATTAACTCATTAAAGTCAATGACAGGTCATG
CTTTAGCTGCCGCTAATGCGATCGAGTCTGTAGCGTTATGTCTGGAAATAGAAAAGCAATATA
TTCATCCAACAATTAATTATCAAACGCCGGACCCTGATTGCGATTTAGATTATATTCCTAATC
AAGGTTGCGCATATCCAATTAAGACCGCATTAAAATTATCGAGTGGTTTTTCTGGTATTCACA
GTGTTATTGTTATGAGGGCAGTAGACAATGCGTAAAAGAGTTGTTGTTACCGGCGTTGGCGCA
GTACATCCTGATGGCAATGATGTCACCGCTATAAAAACAAAAGTGATTCAGAAATTATTGGGT
CAGGAATCGATAAATAATACCAACAAAAGTTCTGTAATAAGGACATTGAATGATTTCGATGGG
GCAAAATATATCAATAACCGCTTAAGACGTAAAATTGATGAATTTTCAGTTTATGGTATCGTC
GCCGTTGAAATGGCATTAAAAGCGAGCAGATTGGATGTAGATAAGCTTGATCCTAATCGTGTT
GGCATATATGTTGGAAACTGTTTTGGCGGATGGCAGCATATTGAGGATGAAGTTAAAGCGCTC
CATGTTGAAGGCATATCGGGGATGGGACCTTATGTTGCTACGGCATGGTTCCCTGCTGCGCTT
CAAGGGCAATTGTCACTGCTTTATGGTTTTAGTGCGCAATCTAAGACATTTTCCACCTCCGAT
GTAGCAGGGATGCAAGCAATAGGCTATGCGGCTGAAGCGATTTCTAATGGTGTTGCCGAAGTG
ATGTTATGTGGCGCGTCAGAACATCTTTCCAGCCCGTTAGTTAAAAGTTTACTGGAGAAAGAG
TCAAGCCAGAAACACTCTGAGGTTTTTGGCGAAAGACAGCCAGGGGACTTTTCCGAAGGCGCT
GCATTTCTAGTGCTGGAAGAGAGGCAACATGCTTTAGAACGCGGCGCTTCGATATTGTGTGAA

TTAACGGGTTTTGTTGATTATTTTTCACCGGATAAAAATACAAGAAATAACACCTTAGAATAT

ACTGCTGAACTATTCAACCATAATGAGAATGCTGTATTTATTATGGATGGAATATATGATGAT

GAAAAAGAAATAACGAGTAAGGCTTTCTCCAATAAAGAGATAAAAACATCATTTATAAATCTG

AGGCCTTACTTGAATAATCAATTTTCAGTCAGCGGCGTAATTGATTCAGTCCTGGCATCATCA

TTTTTATCAGAAAATAACGGGGATGGAGAACAACAATCTAATAAAATAAATGAACTTTCAAAT

ACTAACCAAATAATAATTCAGCGCTTTAGTAACCAGGGTCATGTATGTGCGTTGAGTTTTTCA

GCAATTTAAtctctaaaatatttaattacgcgaggaaaaatatATGAATAATAACCCAGAAGT

AAAAATAAAAACGATTTTGTCTCTTTTTCTTAACGTTAATATTGATGATTTCAATATGGATGC

AAACCTTGCTGATGCCTATGATATGGATTCTACGGAATTGGCTGACTTGGCAAAAGAGATTAC

GAAAGAGTTCGGTATTTCCGTGACGAAAAGTCAGTTCAGTCATTGGGAAACAGGAAGAGCCGT

TCTTGATTTCGTCTCATCAAGTTTAAACGATAAAAATTAA

The underlined portion represents a portion where reading frames of *antD* and *antE* overlap (i.e. the start codon and the stop codon overlap), and the bold lowercase letters at the end represent the sequence between *antE* and *antF.*
**14. *antH* from *Photorhabdus luminescens* (SEQ ID NO: 50)** 
ATGGACGATATTTCTTTATCATCTGATTTTTTTGATCTTTGGATTATCAAAATCGACGATATT

GATTTAGCTTCTATTGAACAGTTAATTCACTGTTCTGATATAGTTCGCCATAACCAAATTTGT

TTAGCGGATAGAAGAAAGAGATTTATATTTAGACGGGCTGCATTACGTTATGTTTTGAGTCAA

TATTTATCTGATTATGAAATCATAACGAATGATAACGGAAAACCTTATATATCCACGGAGCAA

GACTTCAAATATTATTTTTCACTGAGTGCTTCAGGAAACTATTGTGCCATTGGTTTTAGCTCA

AGGGAAATAGGTGTTGATATTGAAGTCACTCCTTCTAAGGTAAAATTTTCAGAAATTATTGAA

CGTTTTATTAAGGATAAAGATTTGGAATATATGAAAGGTATAATGTTAAAACAACTATCAGGA

GTTAGTCTCGGATTTAATAACTATTATCATTTAATGTCATTATATTATTGGGTTAGACTTGAA

GCATATATTAAATTATTTGCTTCGACTTTACATGAGAAATTATTGGTTAATAACTCTGATTCT

GTTAAAGATATGAAAGAATTGGAGGCAAGCACATTATTGATTCATAGTCAGCAATTTGTTTGT

GCCTTATCTCAAAAGAAAGTCATTTCTACACCAAATATCAAGGAAATAAATTATTCCGAAATT
 ATAAGGAACAAAGATGAGTAA
**15. *antG* from *Photorhabdus luminescens* (SEQ ID NO: 51)** 
ATGAAACTAATCTCTATGTTGTTACATTCAGAGCATGATAACTTACATCATGATTGTATTGTC
ACTAAGGATTATCATTATACAAGAAAAGAGGTGATATCTTCTGTTTCCCATTTAATTGATGAT
TTATTGAGTCGAGGAGTGCAAAAAGGTAATAAAGTCATTGTTATATTTGAACATGATGAATTA
GGTGTTTTCTTTTTGGCTGCCGCCAGTGCTATGGGGTTGCATTTATTAATGCCCTATAATTTA
TCATCAGCGACAATCGATGAATGGATTAATTTTACCAATGAAGTGCAATACGATTTTGTTGTT
TATCTCAAAAAAGATAAACATTTTGTTGGAAAATTAAAAGAAAACAACATTAATGTTATTGAT
ATTTCAGATCATAAGATCAGAGTTAGTGATGATATTGCGGAAATCCCAATGATAACTTATTCT
CCGCAACCTATTGCTAACTTTATTGTCCTGTTCACCAGTGGGAGTACAGGCAAACCAAAAGCC
ATTAGTATTTCAGAATCGTTAGTATGTCGTCGAATTTATTCGGTGACCGAGAAATTAAAATTT
ACGCAAGATGCCAAAATATTCATGTCAGGTTTGTTGAATAATACAACTGGAGTGATTTTTTCT
TTCGGCTCATTATTGCATCAATCAACACTTTTTATACCCGAAGATAGAAATGTAGAGAGATGG
CCTGATTATCTTTCTCGCAATAAAATCACTCATATTATGTTACGCCCAGAATCAATGAAATTA
TTCGTTAAATCGACAGCAGAACTTAATATTGATCTCTCTTGTTTACGGGTGGTTGCTTATGGC
GCTGCGGCGATGCCTCCTAGCGTACTTGAGAAAGGGCGACAATTAATTGGCTGTGAATGGGTG
CAGGGATATGGGTTAAGTGAAACTTATGGTCCTTTCTGTTGGGTGGATGAGCAAGATCATCGT
GATAAAAGATATCTCAATTCAATTTATTGTGTTGGTAAGATTGATAATACATTGGAAGTGGCA
GTTAAACCTATTATAGGTTCATCGGATAATATCGGAGAAATTATACTAAGGGGTAAAAGTATT
ATGGAAGGATATTATGATGTCCTTTCTGGAGAAATAACGCCTCCTGATGAATGGTTTGCCACT
GGTGATCTTGGTTATATAGATGAAGAGGGTTATTTAGTTTTGAAAGGACGTAAGCAAAATACG
TTTATGAGTGCTAACGGACACAGAATTTATCCTGAAGAAATTGAATCTATTTTATCCCGAATA
CCCAATGTGAATGTCGCTACGGTTGTTGGTTTTTCTTTCCATGAAAATGGTGTTGCTATTGAT
CAGCCGGTTGCTTGCATGAGTGGAGAGATATCTAAGAAGTCATTACCTGAAATTGAAGATATT
ATTTCATCATTTTTAATGAGTAAACTCAGTCGAGAAAAATGGCCGGATTGGTTCTATGTTACT
GATGAATGCTTTCCGAAAAGCCATAATGATAAGATATTGAAATCAGAGTTAATTAAATCAATC
GATCCTAAGAAATTATTTACATTGAGGAATCAATAA
**16. *ScoMCAT* from *Streptomyces coelicolor* (SEQ ID NO: 52)** 
Atgctcgtactcgtcgctcccggccagggcgcccagacgcccggcttcctgactgactggctc

gccctccccggtgccgctgaccgcgtcgccgcgtggtcggacgccatcggactcgatctcgcc

cacttcggcaccaaggccgacgcggacgagatccgagacacgtccgtggcccagccgctgctg

gtcgccgccggaatcctgtccgccgcggcactcggtacgcagacatctgtcgctgacgcgacg

ggccccgggttcacccccggcgcggtcgccggacacagcgtcggcgagatcaccgccgccgtc

ttcgcgggcgtcctcgacgacaccgccgcgctgtccctcgtacgccgtcgcggcctggccatg

gccgaggccgcggcggtcaccgagaccggcatgtcggcgctgctcgggggcgaccccgaggtg

agcgtcgcgcacctggagcggctcggcctgaccccggcgaacgtgaacggcgccggtcagatc

gtggcggcgggcaccatggagcagctggccgcgctgaacgaggacaagcccgagggtgtgcgc

aaggtcgtcccgctgaaggtggccggcgcgttccacacccgccacatggcccccgccgtggac

aagctcgccgaggccgccaaggcgctgacgccggccgacccgaaggtgacgtacgtctccaac

aaggacgggcgggccgtcgcctccggcaccgaggtgctggaccggctggtcggccaggtcgcc

aacccggtgcgctgggacctgtgcatggagacgttcaaggagctgggcgtcaccgcgatcatc

gaggtgtgtccgggcggcacgctgaccgggctggccaagcgggcgctgcccggagtgaagacg

ctggccctgaagacccccgacgacctcgacgcggcccgtgagctcgtcgccgagcacacccag

gcctaa
17. *actVA-orf5* from *Streptomyces coelicolor* (SEQ ID NO: 53) 
Atgagcgaggacacgatgacccaggagcggccgtccctgacggcacacgcccgccggatcgcc

gaactcgccgggaagcgggcggccgacgccgaacagcagcgccggctgagccccgacgtcgtc

gacgcggtccttcgagccggtttcgccgcccacttcgtaccggtggcgcacggcggccgggcc

gcgacgttcggggagctggtggagcccgtcgcggtgctcggcgaggcctgtgcctcgaccgcc

tggtacgcctcgctcacggcgagcctcggccggatggccgcctacctgccggacgagggccag

gccgagctgtggtccgacggccccgacgccctgatcgtcggtgccctgatgccgctgggccgg
 
gccgagaagaccccgggcggctggcacgtgtcgggcacctggccgttcgtcagcgtcgtggat

cactccgactgggcgctgatctgcgccaaggtcggcgaggagccgtggttcttcgcggtgccg

cgacaggagtacgggatcgtcgacagctggtacccgatgggtatgcgcggaacgggcagcaac

acgctcgtcctcgacggggtgttcgtgccggatgcgcgggcctgcacccgtgcggccatcgcg

gcaggtctcggtccggatgccgaggcgatctgtcacaccgtgcccatgagggcggtcaacggg

ctggccttcgcactgccgatgctcggcgcggcccgcggggccgcggccgtgtggacctcgtgg

accgccggaagactggccgggccgaccgggcagaacgccgtctcgtcccaggaccgcgtggtg

tacgagcacacgctggcccgggccacgggtgagatcgacgcggcccagctgctgttggagcgg

gtcgcggcggtcgccgacgccggctcggcgaccggcgtactggtcggccgcggggcgcgggac

tgcgccctggcggcggagctgctgaccgccgcgaccgaccggctgttcgcctcggcgggcacc

cgggcacaggcccaggacagcccgatgcagcgcctgtggcgcgatgtgcacgcggcgggcagc

catatcgggctgcagttcgggcccggggcggcgctgtacgccggagagctgttgaggaggagc

aacgatggctga
**18. *actVH* from *Streptomyces coelicolor* (SEQ ID NO: 54)** 
Atggcagccgaccagggaatgctccgggacgccatggcccgggtgccggccggggtggcgctc

gtcaccgcccatgaccgcgggggagtcccgcacggtttcaccgccagttcgttcgtgtccgtc

tcgatggagccgccactggcactggtctgcctggctcgtacggccaactccttcccggtgttc

gacagttgcggcgagttcgcggtgagcgtgctgcgcgaggaccacacggacctggccatgcgc

ttcgcgcgcaagtccgcggacaagttcgcgggcggggagttcgtccgtaccgcgcggggagcg

accgtgctcgacggagcggtcgcggtcgtcgagtgcacggtccacgagcgctacccggcgggc

gaccacatcatcctgctcggcgaggtccagtccgtgcacgtcgaggagaagggcgtaccggcg

gtctacgtggaccgccggttcgccgccctgtgctcggcggcgggtgcctgcccgtccgccacc

gggcggggcgtgcccgcgcatgccggctaa
**19. *pobA* from *Pseudomonas fluorescens* (SEQ ID NO: 55)** 
ATGAAAACGCTAAAAACCCAAGTCGCCATTATTGGCGCCGGTCCCTCCGGATTGCTGCTCGGC

CAGTTACTGCACAACGCGGGTATCCAGACCCTGATTCTAGAGCGCCAGAGCGCCGACTACGTG

CAAGGCCGCATCCGTGCCGGGGTGCTGGAGCAAGGCATGGTCGACCTGCTGCGCGAAGCGGGC
 
GTCAGCCGACGCATGGACGCCGAGGGCCTTGTGCATGACGGTTTCGAATTGGCACTCAATGGC
GAACTCACCCACATCGACCTCAAGGCGCTCACCGGCGGCCAGTCGGTGATGATCTACGGCCAG
ACCGAAGTCACCCGTGACTTGATGGCCGCCCGCGAAGCGGCGGGTGGCATCACTCTATACGAA
ACGCAGAACGTGCAGCCTCATGGTCACAAAACTGATCGACCCTGGCTGACCTTCGAGCACCAG
GGTGAAGCTTTTCGCCTGGAGTGCGACTACATCGCGGGCTGTGATGGTTTTCACGGTGTGGCG
CGGCAGTCGATTCCGGCGCAGTCGTTGAAGGTCTTCGAGCGCGTCTATCCCTTCGGTTGGCTG
GGCGTCCTCGCCGACACACCGCCGGTGCATGACGAACTGGTGTACGCCAAACATGCGCGTGGC
TTTGCCCTGTGCAGCATGCGCTCGCCGACCCGCAGCCGCTATTACCTGCAAGTGCCGGTTGAA
GAAGCGCTGGATGAATGGTCGGATCAGCGCTTCTGGGATGAGCTGAAAACCCGTTTGCCCAGT
GCACTGGCGGCCCAACTGGTCACCGGGCCATCCATCGAGAAGAGCATCGCGCCGCTGCGCAGC
TTTGTGGTCGAGCCGATGCAATACGGGCGCCTGTTCCTGCTGGGGGACGCCGCGCATATCGTG
CCGCCCACCGGGGCCAAGGGCTTGAACCTGGCGGCCAGCGACGTGAGTACGCTGTTTCGGATC
TTGCTCAAGGTCTATCGCGAGGGGCGGGTGGACCTGCTGGAACAGTACTCAGCGATCTGCTTG
CGCCGCGTATGGAAAGCCGAACGGTTTTCCTGGTGGATGACTTCGATGTTGCACCAGTTTCCG
GAGGCCGACGGGTTCAGCCAGCGCATTGCCGAGAGCGAGCTTGCGTATTTCATCAGCTCCGAG
GCGGGCCGCAAAACCATCGCAGAAAATTACGTCGGGCTTCCTTACGAAGCTATCGAATAA
**20. *dnrF^{P217K}* from *Streptomyces peucetius* (final dnrF mutant) (SEQ ID NO: 56)** 
gtggccttgacgaagccggatgtcgatgtcctcgtggtgggcggcggtctcggggggctgtcc

accgccctgttcctcgcccgccggggggcgcgggtcctgctggtggagcggcatgccagcacc

tcggtcctgcccaaggcggcaggccagaacccgcgcaccatggaactgttccgcttcggcggc

gtggccgacgagatcctggccacggacgacatccgcggcgcccagggcgacttcaccatcaag

gtcgtggagcgcgtgggcggtcgcgtcctgcacagcttcgcggagagcttcgaggaactggtc

ggtgcgacggaacagtgcacgcccatgccctgggcgctcgctccccaggaccgggtggagccc

gtcctggtggcccacgccgccaagcacggcgcggagatccggttcgccaccgaactgacctcc

ttccaggcgggcgacgacggtgtcacggcccgcctgcgcgacctgggcacgggagcggagagc
 
accgtgagcgcccgctacctggtcgccgccgacggaccccgcagcgcgatccgggagagcctg

ggcatcacccggcacggtcacggcaccctggcccacttcatgggcgtcatcttcgaggccgac

ctcaccgccgtcgtaccgAAGgggtccaccggctggtactacctgcagcacccggacttcacc

ggcacgttcggccccaccgaccggcccaaccggcacaccttctacgtccgctacgaccccgaa

cgcggcgagaggccggaggactacacaccgcagcgctgcaccgagctgatccggctggctgtc

gacgcgcccgggctcgtcccggacatcctcgacatccaggcctgggacatggcggcgtacatc

gccgaccggtggcgcgaagggccggtgctgctggtcggcgatgccgccaaggtcaccccgccc

accgggggcatgggcggcaacaccgccatcggcgacgggttcgacgtggcctggaagctggcc

gccgtgctgcgcggcgaggcgggcgagcggctcctcgacagctacggggcggagcggtcgctc

gtgtcccgcctcgtcgtcgacgagtcactcgccatctacgcccagcgcatggctccccacctg

ctcggcagcgttcccgaggaacgcggtacggcgcaggtcgtcctgggcttccgctaccgctcc

accgccgtcgccgccgaggacgacgaccccgagccgaccgaggatccgcgacgcccgtccggg

cgccccggcttccgcgcaccccacgtctggatcgaacaggacggcacacggcgttccaccgtc

gagttgttcggcgactgctgggtgctcctggccgcaccggagggcggcgcctggggccaggcg

gccgcccgcgccgccgcggatctgggcgtccgcctcgacgtccatctcgtcggccgcgatgtc

gccgccccctccggcgaactgacgcggacctacgggatcggccgggcgggggccagcttggtg

cgcccggacggcgtggtcgcctggcgtacggcagtagcgccgggagcggaggcccaggaccag

ctgagcaccctgctcacccggctgctggcccgctga
**21. *GtCGT*^{*V93Q*/*Y193F*} from *Gentiana triflora* (final GtCGT mutant) (SEQ ID NO: 57)** 
atggggagtttgactaacaacgataatcttcatatttttcttgtgtgcttcatcggccagggc

gtggtcaatcccatgttacgtttggggaaggcgttcgcctccaaagggttacttgtcacttta

agcgcaccggaaatcgttggaactgagatccgtaaggcgaataaccttaatgatgaccaacca

atcaaggtgggttccgggatgattcgtttcgaatttttcgacgatggatgggaatccgtaaac

ggtagcaaaccgtttgacgtatggCAAtacatcaatcacttagaccagacaggccgtcaaaaa

cttccgattatgttaaagaaacatgaggagacagggactcctgtatcttgcttgatcctgaat
 
cccttagtcccttgggtcgcggacgtagccgattcacttcagatcccctgcgctaccttgtgg

gtccaatcttgtgcaagtttttcagcatattaccactaccaccacgggttagtgcctttccca

accgaatcagagcccgagatcgacgtacaacttcctgggatgccacttttgaaatatgatgaa

gtgcccgactTcctgcatccgcgcacaccctaccccttttttggcacgaacattttaggtcaa

ttcaagaatttatccaagaacttctgtatcctgatggataccttctacgagttggaacacgag

atcatcgataatatgtgtaaattgtgtccgattaagccaattggcccgttgtttaagattccg

aaagacccaagctccaacggaatcacgggtaatttcatgaaagtggatgactgcaaggagtgg

ctggacagccgtccaacatcaactgtggtttacgttagtgtcgggtctgttgtatatttgaag

caggagcaggttacagaaatggcatacggcattttaaattcggaagtttcgtttttgtgggtg

ctgcgcccgccgagcaaacgcatcggtacggaaccgcatgtactgcccgaggagttctgggag

aaggccggagatcgtggcaaggtggtgcaatggtcaccccaggagcaggtgcttgctcacccc

gccactgtcggttttttaacacactgtggatggaatagcactcaagaggcgatttcgagcgga

gtgcccgtcatcactttcccacaatttggggaccaagtgaccaatgctaagttccttgtggag

gaatttaaggtcggggtccgtttaggccgcggagagttagaaaatcgcatcatcacacgcgac

gaagtagaacgcgctttacgcgagattacttcaggccccaaggctgaagaggtaaaagagaac

gccttaaaatggaagaagaaggcagaagagacagtagctaaaggcggctactccgaacgtaat

cttgtaggcttcattgaagaggtggctcgtaagactggtacaaagtaa
**22. *ALS* from *Rheum palmatum* (SEQ ID NO: 58)** 
atggcagatgtcctgcaggagatccgcaactcgcagaaggcgagcgggcccgccacggtgctc

gccatcggcactgcccatccaccgacgtgctaccctcaggccgactaccccgacttctacttc

cgagtttgcaagagcgagcacatgaccaaactcaagaagaaaatgcaattcatttgtgacaga

tcggggataaggcagcggtttatgttccacacggaagagaacctggggaagaacccggggatg

tgcacattcgacgggccatcgctgaacgcgcggcaggacatgctgatcatggaagtgccgaag

ctgggggcggaggcggcggagaaggcgatcaaggagtgggggcaggacaagtcccggatcacc

cacctcatcttctgcaccaccacgagcaacgacatgcccggggcggactaccagttcgccacc

ctgttcgggctgaaccccggcgtgagccgcaccatggtctaccagcagggctgcttcgccggg

ggcaccgtgctgcgcctggtcaaggacatcgcggagaacaacaagggggcgcgcgtgctggtg
 
gtgtgctcggagatcgtggccttcgccttccgcgggccccacgaggaccacatcgactccctc

atcgggcagctcctgttcggggacggggccgccgccctcgtggtcgggacagacatcgacgag

agcgtcgagaggcccatcttccagatcatgtcggcgacccaggcgaccatccccaactcgctg

cacaccatggctctccatctgacggaggcggggctgaccttccatctcagcaaggaggtgccc

aaggtggtgagcgacaacatggaggagctcatgctcgaggccttcaagccgctcgggataacc

gattggaactccatattctggcaagtgcatcccgggggtagagccatccttgacaagatcgag

gagaagctggagctcaccaaggataagatgcgggattcccgctacatcttgagcgagtacggg

aatctcaccagcgcctgtgtgctctttgtcatggacgagatgaggaagaggtccttccgggaa

gggaagcagaccaccggagacggctacgagtggggtgtcgccatcggattggggcccggtctt

accgtcgagaccgttgtcttgcgtagcgtccccattccctaa
**23. *accBC* from *Corynebacterium glutamicum* (SEQ ID NO: 59)** 
gtgtcagtcgagactaggaagatcaccaaggttcttgtcgctaaccgtggtgagattgcaatc

cgcgtgttccgtgcagctcgagatgaaggcatcggatctgtcgccgtctacgcagagccagat

gcagatgcaccattcgtgtcatatgcagacgaggcttttgccctcggtggccaaacatccgct

gagtcctaccttgtcattgacaagatcatcgatgcggcccgcaagtccggcgccgacgccatc

caccccggctacggcttcctcgcagaaaacgctgacttcgcagaagcagtcatcaacgaaggc

ctgatctggattggaccttcacctgagtccatccgctccctcggcgacaaggtcaccgctcgc

cacatcgcagataccgccaaggctccaatggctcctggcaccaaggaaccagtaaaagacgca

gcagaagttgtggctttcgctgaagaattcggtctcccaatcgccatcaaggcagctttcggt

ggcggcggacgtggcatgaaggttgcctacaagatggaagaagtcgctgacctcttcgagtcc

gcaacccgtgaagcaaccgcagcgttcggccgcggcgagtgcttcgtggagcgctacctggac

aaggcacgccacgttgaggctcaggtcatcgccgataagcacggcaacgttgttgtcgccgga

acccgtgactgctccctgcagcgccgtttccagaagctcgtcgaagaagcaccagcaccattc

ctcaccgatgaccagcgcgagcgtctccactcctccgcgaaggctatctgtaaggaagctggc

tactacggtgcaggcaccgttgagtacctcgttggctccgacggcctgatctccttcctcgag

gtcaacacccgcctccaggtggaacacccagtcaccgaagagaccaccggcatcgacctggtc
 
cgcgaaatgttccgcatcgcagaaggccacgagctctccatcaaggaagatccagctccacgc

ggccacgcattcgagttccgcatcaacggcgaagacgctggctccaacttcatgcctgcacca

ggcaagatcaccagctaccgcgagccacagggcccaggcgtccgcatggactccggtgtcgtt

gaaggttccgaaatctccggacagttcgactccatgctggcaaagctgatcgtttggggcgac

acccgcgagcaggctctccagcgctcccgccgtgcacttgcagagtacgttgtcgagggcatg

ccaaccgttatcccattccaccagcacatcgtggaaaacccagcattcgtgggcaacgacgaa

ggcttcgagatctacaccaagtggatcgaagaggtttgggataacccaatcgcaccttacgtt

gacgcttccgagctcgacgaagatgaggacaagaccccagcacagaaggttgttgtggagatc

aacggccgtcgcgttgaggttgcactcccaggcgatctggcactcggtggcaccgctggtcct

aagaagaaggccaagaagcgtcgcgcaggtggtgcaaaggctggcgtatccggcgatgcagtg

gcagctccaatgcagggcactgtcatcaaggtcaacgtcgaagaaggcgctgaagtcaacgaa

ggcgacaccgttgttgtcctcgaggctatgaagatggaaaaccctgtgaaggctcataagtcc

ggaaccgtaaccggccttactgtcgctgcaggcgagggtgtcaacaagggcgttgttctcctc

gagatcaagtaa
**24. *accD1* from *Corynebacterium glutamicum* (SEQ ID NO: 60)** 
atgaccatttcctcacctttgattgacgtcgccaaccttccagacatcaacaccactgccggc

aagatcgccgaccttaaggctcgccgcgcggaagcccatttccccatgggtgaaaaggcagta

gagaaggtccacgctgctggacgcctcactgcccgtgagcgcttggattacttactcgatgag

ggctccttcatcgagaccgatcagctggctcgccaccgcaccaccgctttcggcctgggcgct

aagcgtcctgcaaccgacggcatcgtgaccggctggggcaccattgatggacgcgaagtctgc

atcttctcgcaggacggcaccgtattcggtggcgcgcttggtgaggtgtacggcgaaaagatg

atcaagatcatggagctggcaatcgacaccggccgcccattgatcggtctttacgaaggcgct

ggcgctcgcattcaggacggcgctgtctccctggacttcatttcccagaccttctaccaaaac

attcaggcttctggcgttatcccacagatctccgtcatcatgggcgcatgtgcaggtggcaac

gcttacggcccagccctgaccgacttcgtggtcatggtggacaagacctccaagatgttcgtt
 
accggcccagacgtgatcaagaccgtcaccggcgaggaaatcacccaggaagagcttggcgga

gcaaccacccacatggtgaccgctggcaactcccactacaccgctgcgaccgatgaggaagca

ctggattgggtacaggacctggtgtccttcctcccatccaacaatcgctcttacacaccactg

gaagacttcgacgaggaagaaggcggcgttgaagaaaacatcaccgctgacgatctgaagctc

gacgagatcatcccagattccgcgaccgttccttacgacgtccgcgatgtcatcgaatgcctc

accgacgatggcgaatacctggaaatccaggcagaccgcgcagaaaacgttgttattgcattc

ggccgcatcgaaggccagtccgttggatttgttgccaaccagccaacccagttcgctggctgc

ctggacatcgactcctctgagaaggcagctcgcttcgtccgcacctgcgacgcgtttaacatc

ccaatcgtcatgcttgtcgacgtccccggcttccttccaggcgcaggccaggagtatggtggc

atcctgcgtcgtggcgcaaagctgctctacgcatacggcgaagcaaccgttccaaagattacc

gtcaccatgcgtaaggcttacggcggagcgtactgcgtgatgggttccaagggcttgggctct

gacatcaaccttgcatggccaaccgcacagatcgccgtcatgggcgctgctggcgcagtcgga

ttcatctaccgcaaggagctcatggcagctgatgccaagggcctcgataccgtagctctggct

aagtccttcgagcgcgagtacgaagaccacatgctcaacccgtaccacgctgcagaacgtggc

ctgatcgacgccgtgatcctgccaagcgaaacccgcggacagatttcccgcaaccttcgcctg

ctcaagcacaagaacgtcactcgccctgctcgcaagcacggcaacatgccactgtaa
**25. GtCGT^{V93Q/Y193F} (GtUF6CGT1^{V93Q/Y193F}) variant (SEQ ID NO: 61)** 
MGSLTNNDNLHIFLVCFIGQGVVNPMLRLGKAFASKGLLVTLSAPEIVGTEIRKANNLNDDQP
IKVGSGMIRFEFFDDGWESVNGSKPFDVWQYINHLDQTGRQKLPIMLKKHEETGTPVSCLILN
PLVPWVADVADSLQIPCATLWVQSCASFSAYYHYHHGLVPFPTESEPEIDVQLPGMPLLKYDE
VPDFLHPRTPYPFFGTNILGQFKNLSKNFCILMDTFYELEHEIIDNMCKLCPIKPIGPLFKIP
KDPSSNGITGNFMKVDDCKEWLDSRPTSTVVYVSVGSVVYLKQEQVTEMAYGILNSEVSFLWV
LRPPSKRIGTEPHVLPEEFWEKAGDRGKVVQWSPQEQVLAHPATVGFLTHCGWNSTQEAISSG
VPVITFPQFGDQVTNAKFLVEEFKVGVRLGRGELENRIITRDEVERALREITSGPKAEEVKEN
ALKWKKKAEETVAKGGYSERNLVGFIEEVARKTGTK
**26. *zhuIJ* - Codon optimization for *E. coli* (SEQ ID NO: 62)**

atgcgtcatgtagagcatacagtcaccgttgcggccccagcagacttggtttgggaggtactt

gccgatgtcttaggctatgctgacatcttcccaccgacggaaaaagttgaaattcttgaggag

gggcaaggataccaggtagtgcgccttcacgtcgatgttgcgggtgagattaatacatggacc

agtcgtcgcgatttagaccctgcgcgccgcgtaattgcttaccgccaacttgagacggctccg

atcgtgggccacatgagcggggaatggcgtgctttcacactggatgccgaacgtacccaatta

gtcctgactcacgatttcgtaacccgtgcagccggggatgacggtttagtcgccggaaaattg

accccagatgaggcgcgcgaaatgttagaagcggtggtagaacgtaactctgtcgccgactta

aacgcggtccttggagaagctgagcgtcgcgtccgcgcagccggtggagttggtaccgtaact

gcgtaataataattttgtttaactttaagaaggagatatatccatgtcagggcgcaaaacctt

tttagacttaagttttgctacccgcgacacaccgtcggaggcgactccggtggtggtagattt

gctggaccacgtaactggagccaccgtattaggattatcacctgaggatttccccgatggtat

ggctatttccaatgagaccgttacgttgacgacccacactggcacgcacatggatgcgccact

gcactatggtcccttaagtgggggagttccggcaaagtcgattgaccaagtgcccttggaatg

gtgctatggacctggagttcgtttggatgttcgccacgtgccggcaggagatggtattactgt

cgatcatttgaacgccgcgttggatgcagcagagcacgatttggcccccggtgacattgtgat

gctgtggaccggcgcggacgctctgtggggaacccgcgaatacttgagcacgtttccggggtt

aactgggaaggggacacaatttttggtcgaggcgggtgttaaagtcattggcattgatgcatg

gggactggatcgcccgatggcagctatgatcgaagaataccgtcgtacgggcgataaaggagc

attatggccggctcacgtctatggacgcacacgcgaatacctgcaattagagaagcttaataa

tttgggcgctttaccaggagctacagggtatgacatttcatgctttccggttgcggttgcagg

cactggagctgggtggactcgtgtggtcgccgttttcgagcaagaggaagaggattaataa

### Description of abbreviations

- FK:: flavokermesic acid
- KA:: kermesic acid
- CA:: carminic acid

### [Industrial Applicability]

According to the present invention, a C-glycosyltransferase variant has increased ability to form a glycosidic bond compared to wild-type C-glycosyltransferase, thereby enhancing glycoside production effects of polyketides and similar natural products, particularly type I, II, and III polyketides, non-ribosomal peptides, phenylpropanoids, and other aromatic natural products. Therefore, the C-glycosyltransferase variant according to the present invention can be useful in the manufacture of drugs, food additives, nutritional supplements, etc. containing, as a constitutive ingredient, a C-glycoside compound that is increased in amount through polyketide glycoside production of natural products.

### [Sequence List Free Text]

An electronic file is attached.

## Claims

1. A C-glycosyltransferase variant comprising a mutation in at least one amino acid selected from the group consisting of F17, V93, V132, Y193, L164, and R322 in a C-glycosyltransferase represented by SEQ ID NO: 1.

2. The C-glycosyltransferase variant according to claim 1, wherein the variant further comprises a mutation in at least one amino acid selected from the group consisting of F17, V405, P107, L208, L164, P45, I305, L316, F401, Y94, N57, Y187, C16, P319, F167, V132, N206, R406, Q386, V129, L125, L194, I95, S215, L184, Y158, L29, L27, F202, H159, S370, H365, V329, M301, V315, V190, C366, W80, L58, Q210, F312, D61, I207, L363, P196, L106, V93, A394, W314, S155, P88, D99, Y284, E189, G49, H328, E399, T392, F387, A44, P199, E46, R28, V285, I124, R419, L306, Y157, Y200, E373, P191, L214, S376, V15, E332, E51, I417, L98, I323, H161, T383, P127, E309, N84, L313, Q104, T371, N213, G79, L330, N307, K105, L128, A152, I18, N59, W147, S86, L293, E296, S377, L185, K216, F89, S286, F396, F211, Y303, D223, R415, N96, V22, S153, F154, D192, Y193, H195, P201, Y292, and R322 in the C-glycosyltransferase represented by SEQ ID NO: 1.

3. The C-glycosyltransferase variant according to claim 1, wherein the mutation in the amino acid comprises a mutation in amino acids V93 and Y193.

4. The C-glycosyltransferase variant according to claim 1, which comprises substitution of at least one amino acid selected from the group consisting of F17G, V93Q, V132A, Y193F, L164G, and R322D.

5. The C-glycosyltransferase variant according to claim 3, which comprises substitution of amino acids V93Q and Y193F.

6. The C-glycosyltransferase variant according to claim 2, further comprising substitution of at least one amino acid selected from the group consisting of F17G, V405M, P107G, L208G, L164G, P45G, I305A, L316G, F401H, Y94G, N57G, Y187A, C16G, P319G, F167G, V132A, N206E, R406G, Q386H, V129A, L125V, L194A, I95G, S215D, L184G, Y158T, L29A, L27A, F202S, H159G, S370A, H365G, V329T, M301W, V315A, V190A, C366G, W80Y, L58E, Q210G, F312G, D61G, I207P, L363G, P196G, L106G, V93G, A394G, W314C, S155A, P88D, D99G, Y284H, E189A, G49TH328G, E399D, T392A, F387T, A44G, P199E, E46G, R28G, V285I, I124T, R419A, L306M, Y157T, Y200L, E373A, P201G, P191G, L214A, S376G, V15G, E332P, E51C, I417L, L98G, I323A, H161G, T383C, P127A, E309N, N84S, L313T, Q104D, T371A, N213L, G79S, L330G, N307A, K105G, L128D, A152G, S153G, I18A, N59V, W147F, S86V, L293V, E296D, S377A, L185V, K216R, F89A, S286C, F396L, F211G, Y303A, D223G, R415L, N96A, V22H, V93Q, V93L, S153C, F154L, D192S, Y193F, H195Y, H195L, P201T, Y292H, Y292F, R322D, and R322A.

7. The C-glycosyltransferase variant according to claim 4, further comprising substitution of at least one amino acid selected from the group consisting of I18P, Q20M, T50N, T50Q, T50K, T50R, T50V, I95M, I95T, V290G, V290A, I323S, I323A, I95L, V22A, L29A, E46G, V48G, E51C, A55S, S86V, D99G, R103V, C151G, L184G, L194A, E332P, I18A, and P385A.

8. The C-glycosyltransferase variant according to claim 7, further comprising substitution of at least one amino acid selected from the group consisting of I323S, T50R, T50V, I18P, I95T, Q20M, I323A, P385A, L194A, and V48G.

9. A nucleic acid encoding the variant according to any one of claims 1 to 8.

10. A recombinant microorganism into which the nucleic acid according to claim 9 is introduced.

11. The recombinant microorganism according to claim 10, wherein expression of a gene encoding UTP-glucose-1-phosphate uridyltransferase, phosphoglucomutase, and/or nucleoside-diphosphate kinase is enhanced in the recombinant microorganism.

12. The recombinant microorganism according to claim 10, wherein the recombinant microorganism is used for production of a polyketide glycoside and/or a phenylpropanoid glycoside.

13. The recombinant microorganism according to claim 12, wherein a polyketide synthase or phenylpropanoid synthetase is further introduced into the recombinant microorganism.

14. The recombinant microorganism according to claim 12, wherein expression of a pabA gene is attenuated in the recombinant microorganism.

15. The recombinant microorganism according to claim 12, wherein the polyketide is selected from the group consisting of:
type I polyketide selected from the group consisting of rapamycin, lovastatin, erythromycin, rifamycin, avermectin, geldanamycin, ivermectin, calicheamicin, epothilone, triacetic acid lactone, and 6-methylsalicylic acid;
type II polyketide selected from the group consisting of actinorhodin, doxorubicin, daunorubicin, oxytetracycline, SEK4, SEK4b, SEK34, SEK15, SEK26, FK506, DMAC, aklavinone, aklanonic acid, epsilon-rhodomycinone, doxycycline, anthramycin, tetracenomycin, carminic acid, and frenolicin; and
type III polyketide selected from the group consisting of aloesin, aloenin, barbaloin, 5,7-dihydroxy-2-methylchromone, and aloesone, and
the phenylpropanoid is selected from the group consisting of non-ribosomal peptides comprising actinomycin, bacitracin, daptomycin, vancomycin, teixobactin, tyrocidine, gramicidin, zwittermicin A, bleomycin, ciclosporin, pyoverdine, enterobactin, myxochelin A, indigoidine, and cyanophycin, pinocembrin, dihydrokaempferol, eriodictyol, dihydroquercetin, coniferyl alcohol, silybin, isosilybin, silychristin, silinide, 2,3-dehydrosilybin, silydianin, daidzein, genistein, apigenin, luteolin, kaempferol, quercetin, catechin, pelargonidin, cyanidin, afzelechin, myricetin, fisetin, galangin, hesperetin, tangeritin, delphinidin, epicatechin, chrysin, resveratrol, and naringenin.

16. The recombinant microorganism according to claim 12, wherein at least one gene selected from the group consisting of (i) a gene encoding type II polyketide synthase, (ii) a gene encoding 4'-phosphopantetheinyl transferase, (iii) a gene encoding cyclase, (iv) a gene encoding acetyl-CoA carboxylase, and (v) a gene encoding aklavinone 12-hydroxylase is introduced, and the polyketide glycoside is carminic acid.

17. The recombinant microorganism according to claim 16, wherein the gene encoding the type II polyketide synthase is at least one selected from the group consisting of antD (ketosynthase), antE (chain-length factor), antF (ACP), antB (phosphopantetheinyl transferase), and antG (malonyl-CoA:ACP malonyltransferase); or a combination thereof.

18. The recombinant microorganism according to claim 16, wherein the aklavinone 12-hydroxylase comprises a mutation from proline to lysine (P217K) at amino acid position 217 in an amino acid sequence represented by SEQ ID NO: 2.

19. The recombinant microorganism according to claim 16, wherein:
the type II polyketide synthase is derived from P. *luminescens;*
the 4'-phosphopantetheinyl transferase is derived from *Bacillus subtilis* or *P. luminescens;*
the cyclase is derived from *Streptomyces sp.;*
the acetyl-CoA carboxylase is derived from *Corynebacterium glutamicum;* and/or
the aklavinone 12-hydroxylase is derived from *Streptomyces peucetius.*

20. The recombinant microorganism according to claim 12, wherein (i) a gene encoding aloesone synthase is introduced, and the polyketide glycoside is aloesin.

21. The recombinant microorganism according to claim 20, wherein the aloesone synthase is derived from *P. palmatum.*

22. A method of producing a polyketide glycoside and/or a phenylpropanoid glycoside, comprising:
(a) producing a polyketide glycoside and/or phenylpropanoid glycoside by culturing the recombinant microorganism according to claim 10; and
(b) recovering the produced polyketide glycoside and/or phenylpropanoid glycoside.

23. The method according to claim 22, wherein the recombinant microorganism has ability to produce a precursor of the polyketide glycoside and/or the phenylpropanoid glycoside.

24. The method according to claim 22, wherein step (a) comprises culturing the recombinant microorganism according to claim 10 in a medium supplemented with a polyketide and/or a phenylpropanoid.

25. The method according to claim 22, wherein the polyketide glycoside is carminic acid, and in step (a), the microorganism is cultured by adding ascorbic acid to a culture medium during culture.

26. A method of producing a polyketide glycoside and/or a phenylpropanoid glycoside, comprising:
(a) producing a polyketide glycoside and/or phenylpropanoid glycoside by reacting the C-glycosyltransferase variant according to any one of claims 1 to 8 or a microorganism expressing the C-glycosyltransferase variant with a polyketide and/or phenylpropanoid; and
(b) recovering the produced polyketide glycoside and/or phenylpropanoid glycoside.
